(19) **Europäisches Patentamt European Patent Office Office européen des brevets**

(11) **EP 4 375 381 B1**

(12) **EUROPEAN PATENT SPECIFICATION**

(45) Date of publication and mention
of the grant of the patent:
**25.02.2026 Bulletin 2026/09**

(21) Application number: **23210690.6**

(22) Date of filing: **17.11.2023**

(51) International Patent Classification (IPC):
**C12Q 1/6858** $^{(2018.01)}$    **C12Q 1/70** $^{(2006.01)}$

(52) Cooperative Patent Classification (CPC):
(C-Sets available)
**C12Q 1/701; C12Q 1/6858; C12Q 1/70**    (Cont.)

(54) **A METHOD FOR DETERMINING THE PROPORTION OF A LIVE, ATTENUATED DENGUE VIRUS HAVING A NUCLEOTIDE SEQUENCE COMPRISING AT LEAST ONE ATTENUATION LOCUS IN A FORMULATION**

VERFAHREN ZUR BESTIMMUNG DES ANTEILS EINES LEBENDEN ABGESCHWÄCHTEN DENGUE VIRUS MIT EINER NUKLEOTIDSEQUENZ MIT MINDESTENS EINEM ABSCHWÄCHUNGSLOKUS IN EINER FORMULIERUNG

PROCÉDÉ POUR DÉTERMINER LA PROPORTION VIRUS DE LA DENGUE VIVANT ATTÉNUÉ AYANT UNE SÉQUENCE NUCLÉOTIDIQUE COMPRENANT AU MOINS UN LOCUS D'ATTÉNUATION DANS UNE FORMULATION

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **18.11.2022 EP 22208356**

(43) Date of publication of application:
**29.05.2024 Bulletin 2024/22**

(73) Proprietor: **Takeda Vaccines, Inc.
Cambridge, MA 02139 (US)**

(72) Inventors:
• **MAYER, Dietmar**
  **06861 Dessau-Roßlau (DE)**
• **BORUTZKI, Stefan**
  **06861 Dessau-Roßlau (DE)**
• **RICHTER, Benjamin**
  **06861 Dessau-Roßlau (DE)**

(74) Representative: **Maiwald GmbH
Elisenhof
Elisenstraße 3
80335 München (DE)**

(56) References cited:
EP-A1- 3 620 174        EP-A1- 3 892 737
WO-A1-2014/093182    WO-A1-2022/146773
US-A1- 2014 134 205    US-A1- 2015 197 787

Remarks:
The complete document including Reference Table(s) and the Sequence Listing(s) can be downloaded from the EPO website

(52) Cooperative Patent Classification (CPC): (Cont.)

C-Sets
**C12Q 1/6858, C12Q 2535/122;**
**C12Q 1/70, C12Q 2531/113, C12Q 2535/122**

## Description

### Co-filed sequence listing

The present specification makes reference to a sequence listing (submitted electronically on the same date as the present application). The entire contents of the Sequence Listing are incorporated herein by reference.

### Technical field

The present invention relates to methods for determining the proportion of an attenuated genotype of a live, attenuated Dengue virus having a nucleotide sequence comprising at least one attenuation locus in a formulation as well as quality control methods for the release of vaccine formulations comprising said live, attenuated flavivirus.

### Background of the Invention

Dengue virus is an arbovirus in the *Flaviviridae* family. It is an enveloped virus with an isometric nucleocapsid, consisting of capsid proteins and a linear positive sense RNA genome. Dengue viruses are endemic in most regions of the tropics and are transmitted by mosquitoes, typically *Aedes aegypti,* causing symptomatic infections or asymptomatic seroconversion. Symptomatic dengue infection is a systemic and dynamic disease. It has a wide clinical spectrum that includes both severe and non-severe clinical manifestations. There are four different dengue serotypes (DENV-1 to DENV-4) which are genetically related to yellow fever and similar tick-borne encephalitis viruses.

One tetravalent dengue vaccine (TDV) tested in clinical trials and now approved as the product QDENGA® consists of all four dengue serotypes, TDV-1, TDV-2, TDV-3 and TDV-4. TDV-2 is a live attenuated recombinant virus that originated from PDK-53 that became non-pathogenic after continuous passaging of DENV-2 virus in primary dog kidney (PDK) cells. The mutations necessary and sufficient for the attenuated phenotype of TDV-2 virus have been genetically identified (Butrapet et al., J. Virol., vol. 74, no. 7 (2000), 3011-3019). These mutations are a C-to-T mutation at genomic nucleotide position 57 in the 5' noncoding region, a G-to-A mutation at genomic nucleotide position 2579 (causing a Gly-to-Asp mutation at amino acid position 53 in the nonstructural protein (NS) 1) and an A-to-T mutation at genomic nucleotide position 5270 (causing a Glu-to-Val mutation at amino acid position 250 in the nonstructural protein (NS) 3. These three genetic determinants of attenuation reside outside of the structural gene regions of the TDV-2 viral genome. TDV-1, TDV-3 and TDV-4 are chimeric variants having the backbone of Dengue virus 2 PDK53 and the prM and E genes of Dengue virus 1, Dengue virus 3 and Dengue virus 4, respectively, which maintain the attenuation phenotype of TDV-2.

EP3892737 discloses a PCR-based method for detecting/quantifying at least one attenuated genotype of a live, attenuated TDV, in a virus formulation (i.e. attenuated virus vaccines)

For quality control of the monovalent drug substance comprising a single live, attenuated dengue virus and the tetravalent drug product comprising a mixture of all four live, attenuated viruses it is essential to confirm that essentially all of the viruses contained in the formulation are live, attenuated, i.e. have the attenuated type-nucleotide at the attenuation locus. Only when this has been confirmed, the formulations may be released for distribution.

In view thereof, there is a need for a quantitative method for determining the proportion of an attenuated genotype of a live, attenuated flavivirus in a formulation comprising (i) at least one live, attenuated flavivirus having a nucleotide sequence comprising an attenuated-type nucleotide at at least one attenuation locus. The method shall be suitable for validation. Accordingly, the method shall be specific and robust in the presence of further components in the formulation such as pharmaceutically acceptable excipients. A further technical problem is the provision of a quality control method using the above method for use in pharmaceutical industry.

### Summary of the Invention

The technical problems underlying the invention are solved by the provision of the subject-matter as defined in the claims.

According to a first aspect, a method for determining the proportion of an attenuated genotype of a live, attenuated flavivirus in a formulation comprising (i) at least one live, attenuated Dengue virus having a nucleotide sequence comprising an attenuated-type nucleotide at at least one attenuation locus, and (ii) and optionally at least one further Dengue virus having a nucleotide sequence comprising a nucleotide other than the attenuated-type nucleotide at the at least one attenuation locus is provided, wherein the method comprises the steps:

a) purifying Dengue virus nucleic acids from said formulation;
b) preparing double-stranded DNA templates from said Dengue virus nucleic acids;
c) amplifying said viral nucleic acids of a) or said double-stranded DNA templates of b) by polymerase chain reaction (PCR) by using a primer pair comprising sequences specific for a flavivirus sequence comprising the at least one attenuation locus such that PCR products comprising the attenuation locus are generated;

d) sequencing said PCR products by next generation sequencing by synthesis; and

e) determining the proportion of the attenuated genotype of the live, attenuated Dengue virus in the formulation from the ratio of

(i) the number of sequence reads in which the nucleotide at the at least one attenuation locus is identical to the nucleotide of the live, attenuated Dengue virus at the at least one attenuation locus to

(ii) the total number of sequence reads.

According to a second aspect, the use of the method according to the invention in the quality control of vaccines containing at least one live, attenuated Dengue virus is provided

wherein the live, attenuated dengue virus comprises three attenuation loci at positions 57, 2579 and 5270, wherein the sequence numbering relates to the nucleotide sequence of dengue virus type 2 according to SEQ ID NO: 2 and the primer pair of step c) is selected from the group consisting of:

i) a forward primer comprising SEQ ID NO: 11, or a variant thereof having at least 95% sequence identity and a reverse primer comprising SEQ ID NO: 12, or a variant thereof having at least 95% sequence identity;

ii) a forward primer comprising SEQ ID NO: 13, or a variant thereof having at least 95% sequence identity and a reverse primer pair comprising SEQ ID NO: 14, or a variant thereof having at least 95% sequence identity; and

iii) a forward primer comprising SEQ ID NO: 15, or a variant thereof having at least 95% sequence identity and a reverse primer comprising SEQ ID NO: 16, or a variant thereof having at least 95% sequence identity.

According to a third aspect, a method for the quality control of vaccines comprising at least one live, attenuated Dengue virus comprising performing the method of any one of claims 1 to 14 and performing a quality control step, wherein the vaccine is accepted if the proportion of the at least one live, attenuated virus in the vaccine is at least 90% in the vaccine, preferably at least 95 %, more preferably at least 97% and most preferably at least 99%.

The inventors have found that the present assay is specific and robust in the presence of further components. It has been validated for use in biopharmaceutical industry. As compared to single-molecule real-time (SMRT) sequencing, the present method has the advantages of high-throughput, low error rate and being less expensive.

## Brief Description of the Drawings

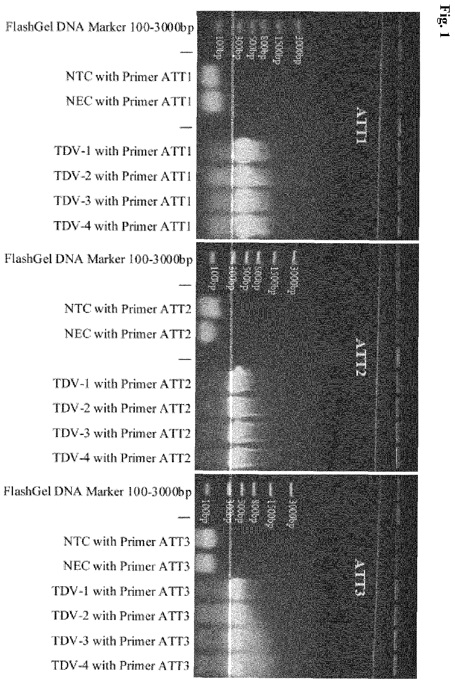

**Figure 1** FlashGel photos of the RT-PCR products (ATT1, ATT2 and ATT3) for the demonstration of the specificity for TDV-1, -2, -3 and -4, with the Primer pairs: ATT1 (324 bp), ATT2 (333 bp) and ATT3 (408 bp). The negative controls were negative, only the residual primers resp. primer dimers (1 primer >50 nt) are visible. ATT stands for attenuated and refers to the three attenuation sites ATT1, ATT2 and ATT3.

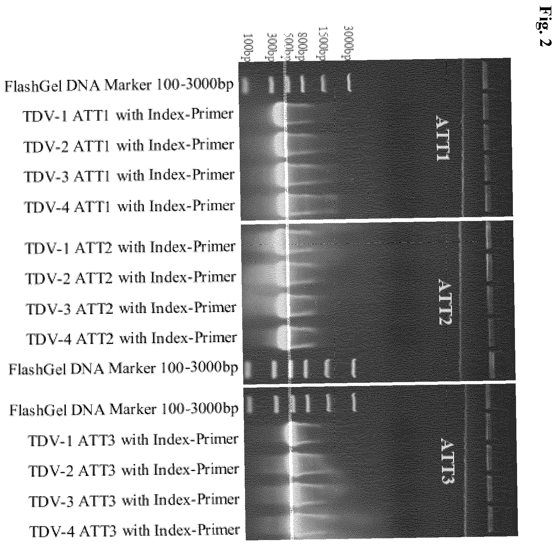

**Figure 2** FlashGel photos of the Index-PCR products for the demonstration of the specificity for TDV-1, -2, -3 and -4, with the different forward and reverse index-primer pairs: ATT1 (393 bp), ATT2 (402 bp) and ATT3 (477 bp).

## Detailed Description of the Invention

Unless defined otherwise, all technical and scientific terms used herein have the same meaning as commonly understood by one of ordinary skill in the art to which the invention pertains. Although, any processes and materials similar or equivalent to those described herein can be used in practice for testing of the present methods the preferred materials and processes are described herein. In describing and claiming the present invention, defined by the appended claims the following terminology will be used in accordance with the definitions set out below.

Where the term "comprise" or "comprising" is used in the present description and claims, it does not exclude other elements or steps. For the present purpose the term "consisting of" is considered to be an optional embodiment of the term "comprising". If hereinafter a group is defined to comprise at least a certain number of embodiments, this is also to be understood to disclose a group which optionally consists only of these embodiments. Where an indefinite or a definite article is used when referring to a singular noun e.g. "a" or "an", "the", this includes a plural form of that noun unless specifically stated.

*Vice versa,* when the plural form of a noun is used it refers also to the singular form. Furthermore, the terms first, second, third or (a), (b), (c) and the like in the description and in the claims are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein unless context clearly indicates otherwise.

In the context of the present disclosure any numerical value indicated is typically associated with an interval of accuracy that the person skilled in the art will understand to still ensure the technical effect of the feature in question. As used herein, the deviation from the indicated numerical value is in the range of ± 10%, and preferably of ± 5%. The aforementioned deviation from the indicated numerical interval of ± 10%, and preferably of ± 5% is also indicated by the terms "about" and "approximately" used herein with respect to a numerical value.

According to the first aspect of the present invention, is defined by appended claims 1-14.

An RNA virus can be characterized by its RNA sequence. It is, alternatively or in addition, also common practice to characterize the genome of RNA viruses by the corresponding DNA sequences and corresponding DNA sequences are readily understood to reflect the RNA genome in the virus. Therefore, reference to "t" or "thymine" in the entire disclosure is to be understood as reference to "u" or "uracil", respectively, if the genomic RNA virus sequence is meant. "Sample" herein means any sample obtained from a vaccinated individual or a Dengue virus infected patient (also referred to herein as a "Dengue disease patient"), or any sample to be tested at the stage of quality control of manufactured vaccines, i.e. a sample containing one to four live, e.g. one, two, three, or four attenuated Dengue virus vaccine strains. If the sample is from a vaccinated individual or Dengue disease patient, it may be from whole blood or serum, most preferably from serum. If the sample is to be tested at the stage of quality control, it may also be a vaccine composition or a stock solution of a vaccine composition prior to administration. In such a scenario, testing according to the inventive method may be important for quality control and safety reasons.

In a preferred embodiment, the method is performed on a vaccine composition or a stock solution of a vaccine composition prior to administration.

"An attenuated virus" herein means that the virus has a reduced replication capacity and/or a reduced infectivity in host cells compared to wild-type virus. The replication capacity and/or infectivity may be determined *in vitro* in suitable cell systems or *in vivo* in suitable animal models. Attenuation may be achieved by serial passaging of the virus in a foreign host such as in tissue culture, embryonated eggs or live animals. Alternatively, attenuation may be performed by chemical agents.

"Live attenuated" viruses are important for use in viral vaccines, since the live attenuated virus generates a stronger immune response compared to an inactivated virus. As used herein, the term "live attenuated virus" can refer to e.g. a chimeric virus or a non-chimeric virus.

In embodiments where the flavivirus composition comprises more than one live, attenuated virus, the composition in a preferred embodiment comprises at least one chimeric live attenuated flavivirus. More preferably, the composition comprises one chimeric flavivirus and three non-chimeric flaviviruses. Particularly preferred, the composition comprises live, attenuated DENV-1, DENV-2 and DENV-4 viruses and a chimeric DENV-2/DENV-4 chimeric virus.

In an alternative preferred embodiment the composition comprises at least two chimeric live, attenuated viruses and at least one non-chimeric flavivirus. More preferably, the composition comprises a live, attenuated DENV-2 virus, a chimeric DENV-2/DENV-1 virus, a chimeric DENV-2/DENV-3 virus and a chimeric DENV-2/DEN-4 virus.

The live attenuated virus may be an RNA virus or a DNA virus. Suitable live attenuated RNA viruses may be selected from dengue virus, poliovirus, rubella virus, measles virus, yellow fever virus, mumps virus, zika virus, and influenza virus. Preferably, the live attenuated RNA virus is dengue virus. The live attenuated RNA virus may be a chimeric virus. Suitable live attenuated DNA viruses include varicella zoster virus, vaccinia virus, smallpox virus, Herpes simplex virus and chikungunya virus. The live attenuated virus may differ from the wild-type virus in one or more mutations in the nucleic acid sequence of the virus. For example, for one tetravalent dengue vaccine (TDV) it is known that attenuation of neurovirulence in newborn mice is determined by mutations 5'NCR-57 C->U, NS1-53 Gly->Asp (nt-2579 G->A) and NS3-250 Glu->Val (nt-5270 A->U) (Butrapet et al., J. Virol. 74 (2000), 3011-3019). The above nucleotide sequence numbering relates to the nucleotide sequence of the live attenuated Dengue virus serotype 2 (TDV-2) set forth in SEQ ID NO:2.

The live, attenuated flavivirus can be a flavivirus in which all components are derived from the same flavivirus serotype or it can be a chimeric flavivirus having parts from two or more flavivirus serotypes. The live, attenuated dengue virus can be a dengue virus in which all components are derived from the same dengue serotype or it can be a chimeric dengue virus having parts from two or more dengue serotypes. A "virus strain" and in particular a "dengue virus strain" is a genetic subtype of a virus, in particular of a dengue virus, which is characterized by a specific nucleic acid sequence. A dengue serotype may comprise different strains with different nucleic acid sequences which have the same cell surface antigens and are therefore recognized by the same antibodies. A dengue virus strain can be a dengue virus in which all components are derived from the same dengue serotype or it can be a chimeric dengue virus having parts from two or more dengue serotypes.

A "chimeric virus" or "chimeric strain" or "chimeric virus strain" in general comprises parts from at least two different viruses. For example, a chimeric virus can comprise the prM and E proteins of dengue virus and the other proteins from another flavivirus. The chimeric virus can comprise the prM and E proteins of dengue virus and the other proteins from another flavivirus such as yellow fever virus, Zika virus, West Nile virus, Japanese encephalitis virus, St. Louis encephalitis virus and tick-borne encephalitis virus. The chimeric virus can comprise the prM and E proteins of dengue virus and the other proteins from yellow fever virus strain YF-17D. Such chimeric viruses are present in the commercial product Dengvaxia® and are described in, e.g., WO 98/37911, WO 03/101397, WO 2007/021672, WO 2008/007021, WO 2008/047023 and WO 2008/065315.

A "chimeric dengue virus" or "chimeric dengue serotype strain" or "chimeric dengue strain" preferably comprises parts from at least two different dengue serotypes, i.e. a dengue-dengue chimera. Such chimeric dengue viruses are described in WO 01/060847 A2, WO 2014/150939 A2 and WO 2017/179017 A1 .

As used herein, "TDV" refers to a tetravalent live attenuated dengue vaccine that comprises a mixture of the four live attenuated dengue virus strains TDV-1, TDV-2, TDV-3 and TDV-4 expressing surface antigens from the four dengue serotypes DENV-1 (dengue serotype 1), DENV-2 (dengue serotype 2), DENV-3 (dengue serotype 3) and DENV-4 (dengue serotype 4), respectively.

As used herein, "TDV-2" refers to a molecularly characterized and cloned dengue serotype 2 strain derived from the live attenuated DEN-2 PDK-53 virus strain. The PDK-53 strain is described for example in Bhamarapravati et al. (1987) Bulletin of the World Health Organization 65(2): 189-195. In one embodiment, the TDV-2 strain served as a backbone for the chimeric TDV-1, TDV-3 and TDV-4 strains into which parts from the TDV-1, TDV-3 and TDV-4 strains were introduced.

As used herein, a "TDV-1" refers to a dengue virus chimeric construct which comprises parts from both DENV-2 and DENV-1. Preferably, in TDV-1 the prM and E proteins from DENV-1 replace the prM and E proteins from DENV-2.

As used herein, "TDV-3" refers to a dengue virus chimeric construct which comprises parts from both DENV-2 and DENV-3. Preferably, in TDV-3 the prM and E proteins from DENV-3 replace the prM and E proteins from DENV-2.

As used herein, "TDV-4" refers to a dengue virus chimeric construct which comprises parts from both DENV-2 and DENV-4. Preferably, in TDV-4 the prM and E proteins from DENV-4 replace the prM and E proteins from DENV-2.

In one embodiment, TDV-1 is characterized by the nucleotide sequence according to SEQ ID No 1. In one embodiment, TDV-2 is characterized by the nucleotide sequence according to SEQ ID No. 2. In one embodiment, TDV-3 is characterized by the nucleotide sequence according to SEQ ID No. 3. In one embodiment, TDV-4 is characterized by the nucleotide sequence according to SEQ ID No. 4.

A "vaccine composition" or "vaccine formulation" as used herein means a composition containing antigens of one to four, e.g. one, two, three, or four, of the dengue serotypes and formulated for administration to an individual. Preferably, a vaccine composition comprises a dengue antigen of each of serotypes 1 to 4 which are each independently selected from the group consisting of: (i) a live attenuated non-chimeric dengue virus and (ii) a live attenuated chimeric dengue virus.

In a preferred embodiment, a vaccine composition comprises a dengue antigen of each of serotypes 1 to 4, wherein said dengue antigens of serotypes 1, 3 and 4 are each a live attenuated chimeric dengue virus and said dengue antigen of serotype 2 is selected from the group consisting of a live attenuated dengue virus and a live attenuated chimeric dengue virus. For example, a vaccine composition may comprise a dengue antigen of each of serotypes 1 to 4, wherein said dengue antigens of serotypes 1, 3 and 4 are each a live attenuated chimeric dengue/dengue virus and said dengue antigen of serotype 2 is a live attenuated dengue virus. For example, a vaccine composition may be the tetravalent mixture of dengue antigens of each of serotypes 1 to 4 (referred to as DENVax) which is disclosed in Huang et al., PLoS Negl Trop Dis 7(5): e2243 (2013).

In another preferred embodiment, a vaccine composition may comprise a dengue antigen of each of serotypes 1 to 4, wherein said dengue antigens of serotypes 1, 3 and 4 are each a live attenuated chimeric yellow fever (YF)/dengue virus and said dengue antigen of serotype 2 is a live attenuated dengue virus.

In another preferred embodiment, a vaccine composition comprises a dengue antigen of each of serotypes 1 to 4, wherein said dengue antigens of serotypes 1, 3 and 4 are each independently selected from the group consisting of: (i) a live attenuated dengue virus and (ii) a live attenuated chimeric dengue virus and said dengue antigen of serotype 2 is a live attenuated chimeric dengue virus. For example, a vaccine composition may comprise a dengue antigen of each of serotypes 1 to 4, wherein said dengue antigens of serotypes 1, 3 and 4 are each a live attenuated dengue virus and said dengue antigen of serotype 2 is a live attenuated chimeric dengue/dengue virus. For example, a vaccine composition may be any of the tetravalent mixtures (referred to as TV001 , TV002, TV003 and TV004') of dengue antigens of each of serotypes 1 to 4 which are disclosed in Durbin et al., Journal of Infectious Diseases (2013), 207, 957-965. Preferably, a vaccine composition is TV003.

In another preferred embodiment, a vaccine composition comprises a dengue antigen of each of serotypes 1 to 4, wherein each of said dengue antigens is a live attenuated chimeric dengue virus, preferably a chimeric YF/dengue virus, more preferably a chimeric YF/dengue virus which comprises an attenuated YF genomic backbone whose prM-E sequence has been substituted with the prM-E sequence of dengue virus.

In a preferred embodiment, a live attenuated chimeric dengue virus comprises one or more proteins from a dengue virus and one or more proteins from a different flavivirus. Preferably, the different flavivirus is a yellow fever virus (i.e. a chimeric YF/dengue virus). Preferably a live attenuated chimeric dengue virus comprises an attenuated yellow fever virus genome whose prM-E sequence has been substituted with the prM-E sequence of a dengue virus. Alternatively, a live attenuated chimeric dengue virus comprises one or more proteins from a first dengue virus and one or more proteins from a second dengue virus (i.e. a chimeric dengue/dengue virus). Preferably said first dengue virus and said second dengue virus are of different serotypes. Where said first dengue virus and said second dengue virus are of the same serotype, said first and second dengue viruses are different strains of the same serotype.

In an especially preferred embodiment, the inventive method is performed on a sample that is a vaccine formulation and comprises at least one live, attenuated dengue virus, preferably at least two different live, attenuated dengue viruses, more preferably at least three different live, attenuated dengue viruses, and most preferably at least four different live, attenuated viruses. In one such embodiment, the at least one live, attenuated virus comprises one or more of a nucleotide sequence selected from the group consisting of SEQ ID NOs: 1 to 4 (TDV-1 to TDV-4), or a variant of the foregoing having at least 90% sequence identity. That is, in an embodiment, the inventive method is performed on a sample that is a vaccine formulation and comprises or consists of TDV-1, TDV-2, TDV-3, or TDV-4 or a variant of the foregoing having at least 90% sequence identity. In another embodiment, the inventive method is performed on a sample that is a vaccine formulation and comprises or consists of TDV-1 and TDV-2 or (a) variant(s) of the foregoing having at least 90% sequence identity; TDV-1 and TDV-3 or (a) variant(s) of the foregoing having at least 90% sequence identity; TDV-1 and TDV-4 or (a) variant(s) of the foregoing having at least 90% sequence identity; TDV-2 and TDV-3 or (a) variant(s) of the foregoing having at least 90% sequence identity; TDV-2 and TDV-4 or (a) variant(s) of the foregoing having at least 90% sequence identity; or TDV-3 and TDV-4 or (a) variant(s) of the foregoing having at least 90% sequence identity. In another embodiment, the inventive method is performed on a sample that is a vaccine formulation and comprises or consists of TDV-1, TDV-2, and TDV-3 or (a) variant(s) of the foregoing having at least 90% sequence identity; TDV-1, TDV-2 and TDV-4; TDV-1, TDV-3, and TDV-4 or (a) variant(s) of the foregoing having at least 90% sequence identity; or TDV-2, TDV-3, and TDV-4 or (a) variant(s) of the foregoing having at least 90% sequence identity. In another embodiment, the inventive method is performed on a sample that is a vaccine formulation and comprises or consists of all of TDV-1, TDV-2, TDV-3, and TDV-4 or (a) variant(s) of the foregoing having at least 90% sequence identity. Preferably, in any of the above embodiments, the sequence identity of the variant(s) is at least 90, 91, 92, 93, 94, 95, 96, 97, 98, or 99.5%.

"Purifying virus nucleic acids from the sample" may be performed using any method for DNA or RNA isolation known in the art. Suitable methods comprise *inter alia* steps such as centrifugation steps, precipitation steps, chromatography steps, dialyzing steps, heating steps, cooling steps and/or denaturation steps. Any commercially available kit for the purification of nucleic acids may be used. Where the dengue nucleic acids are RNAs, any known method and/or any commercially available kit for RNA purification may be used.

"Preparing double-stranded DNA templates" herein means that if the virus nucleic acid is an RNA, step b) includes a step of reverse transcription with a reverse transcriptase, primers and a mixture of nucleotides such that a RNA/DNA hybrid is generated which is subsequently converted into a double-stranded cDNA template. The reaction may be carried out in an automated thermal cycler. The reaction may be a one-step RT-PCR also encompassing step c) of the inventive method. Any method known in the art and any commercially available, reverse transcriptase, kit and/or thermal cycler may be used.

Accordingly, in a preferred embodiment of any of the above methods, the virus nucleic acids are isolated virus RNA and step b) comprises a step of reverse transcription of the isolated virus RNA in the presence of a reverse transcriptase using a reverse primer.

If the virus nucleic acid is a single-stranded DNA, the double-stranded DNA-template is generated by the activity of a template-dependent DNA polymerase. Any method known in the art and any commercially available polymerase, kit and/or thermal cycler may be used.

"Amplifying said double-stranded templates from step b)" herein means that the double-stranded DNA templates of step b) are incubated with a primer pair(s) (forward and reverse primers), nucleotides and a template-dependent DNA polymerase such that a PCR product is generated for each instance of a template of one serotype and matching primer pair for that same serotype. The reaction may be carried out in an automated thermal cycler. Any PCR method known in the art and any commercially available polymerase, kit, and/or thermal cycler may be used.

"Amplifying said viral nucleic acids of a)" herein means that the flavivirus nucleic acids of step a) are incubated with the a primer pair(s) (forward and reverse primers), nucleotides and a reverse transcriptase such that a PCR product is generated for each instance of a template of one serotype and matching primer pair for that same serotype. A reverse transcriptase (RT) in an enzyme that uses RNA as a template and synthesizes complementary DNA. The reaction may be carried out in an automated thermal cycler. Any RT-PCR method known in the art and any commercially available polymerase, kit, and/or thermal cycler may be used.

Hence, in a preferred embodiment of any of the above methods, the reaction may be a one-step RT-PCR also encompassing step b) of the inventive method. Hence, in a preferred embodiment steps b) and c) of the inventive methods may be performed simultaneously, i.e. in the same reaction mixture. In the same reaction mixture, the steps may still be performed one after the other. The product of step a) may serve as a template for step c).

In another embodiment, steps b) and c) of the inventive methods may be performed sequentially. When performed sequentially, step b) may be performed before step c). In one embodiment, step b) may be performed by reverse transcriptase. When performed sequentially, the steps b) and c) may be performed in the same reaction mixture. When performed sequentially, the steps b) and c) may be performed in separate reaction mixtures. The product of step b) may serve as a template for step c).

Preferably, the PCR amplification reaction of step c) comprises at least 10 PCR cycles using a DNA polymerase, the inventive primer pairs, and nucleotides. More preferably, 10 to 50 cycles, even more preferred 20 to 45 cycles are used. Most preferably, 35 cycles are used. It is also preferred that the PCR amplification reaction includes a denaturing step at a temperature from about 90°C to about 99°C, an annealing step at a temperature from about 45°C to about 75°C, and an extension step at a temperature from about 60°C to about 75°C, more preferably denaturing is performed at about 96°C, annealing is performed at 60°C and extension is performed at 68°C. In a preferred embodiment, the amplifying step c) is carried out in a thermocycling process at +50°C for 30 min; +96°C for 2 min; and multiple cycles of +96°C for 20 s; +60°C for 45s and +68°C for 45s. In a most preferred such embodiment, the multiple cycles are 35 cycles. Optionally, the reaction may me stopped or stored at a temperature of about 4°C after completion. The reaction may be stored at a temperature of about 4°C, or about -20°C, or about -80°C after completion.

The term "primer" as used herein denotes an oligonucleotide that acts as an initiation point of nucleotide synthesis under conditions in which synthesis of a primer extension product complementary to a nucleic acid strand is induced.

The primers may be generated such that they are able to bind to the sequence to be reverse transcribed or amplified.

Primers useful in the method comprise a specific sequence that can bind to the sequence to be reverse transcribed or amplified.

Specific primers may be used by themselves, or they may form part of a pair of fusion primers. The term "fusion primer" as used herein refers to primers comprising an adapter sequence or overhang sequence and a specific sequence.

The part of the fusion primer sequence that can bind to the sequence to be reverse transcribed or amplified is referred to as the "specific sequence". The specific sequence of the fusion primer may comprise from 5 to 50 nucleotides, preferably, 8 to 30 nucleotides; more preferably the primer comprises 15 to 25 nucleotides. The specific sequences of the fusion primer are complementary to at least a portion of the sequence to be reverse transcribed or amplified. Preferably, the specific sequences of the fusion primer s are fully complementary to a sequence within the sequence to be reverse transcribed or amplified. Methods for the design of sequence specific primers and probes are known in the art.

The forward primer of the primer pair specific for attenuation locus 1 may have 85, 86, 87, 88, 89, 90, 91, 92, 93, 94, 95, 96, 97, 98, 99, 99.5, or 100% sequence identity to SEQ ID NO: 5. In an embodiment, the forward primer with at least 85 sequence identity has the same length as SEQ ID NO: 5.

The reverse primer of the primer pair specific for attenuation locus 1 may have 85, 86, 87, 88, 89, 90, 91, 92, 93, 94, 95, 96, 97, 98, 99, 99.5, or 100% sequence identity to SEQ ID NO: 6. In an embodiment, the reverse primer with at least 85 sequence identity has the same length as SEQ ID NO: 6.

The forward primer of the primer pair specific for attenuation locus 2 may have 85, 86, 87, 88, 89, 90, 91, 92, 93, 94, 95, 96, 97, 98, 99, 99.5, or 100% sequence identity to SEQ ID NO: 7. In an embodiment, the forward primer with at least 85 sequence identity has the same length as SEQ ID NO: 7.

The reverse primer of the primer pair specific for attenuation locus 2 may have 85, 86, 87, 88, 89, 90, 91, 92, 93, 94, 95, 96, 97, 98, 99, 99.5, or 100% sequence identity to SEQ ID NO: 8. In an embodiment, the reverse primer with at least 85 sequence identity has the same length as SEQ ID NO: 8.

The forward primer of the primer pair specific for attenuation locus 3 may have 85, 86, 87, 88, 89, 90, 91, 92, 93, 94, 95, 96, 97, 98, 99, 99.5, or 100% sequence identity to SEQ ID NO: 9. In an embodiment, the forward primer with at least 85 sequence identity has the same length as SEQ ID NO: 9.

The reverse primer of the primer pair specific for attenuation locus 3 may have 85, 86, 87, 88, 89, 90, 91, 92, 93, 94, 95, 96, 97, 98, 99, 99.5, or 100% sequence identity to SEQ ID NO: 10. In an embodiment, the reverse primer with at least 85 sequence identity has the same length as SEQ ID NO: 10.

In a preferred embodiment the final concentration of each of the forward and reverse primer in the amplifying step c) is about 0.1 to about 0.3 $\mu$M. More preferably, the final concentration of each of the forward and reverse primer in the amplifying step c) is about 0.2 $\mu$M.

The "adapter sequence" or "overhanging adapter sequence" of the fusion primer comprises a sequence that can be used for a sequencing process. The adapter sequence of the fusion primer may comprise from 15 to 50 nucleotides, preferably, 25 to 40 nucleotides; more preferably the primer comprises 30 to 35 nucleotides. The adapter sequences for forward and reverse specific primers are different. Hence, each forward fusion primer comprises a forward adapter sequence and a forward specific sequence. Each reverse fusion primer comprises a reverse adapter sequence and a reverse specific sequence.

The forward adapter sequence may have at least 85, 86, 87, 88, 89, 90, 91, 92, 93, 94, 95, 96, 97, 98, 99, 99.5, or 100% sequence identity to SEQ ID NO: 17. In an embodiment, the forward adapter sequence with at least 85 %sequence identity has the same length as SEQ ID NO: 17. The reverse adapter sequence may have at least 85, 86, 87, 88, 89, 90, 91, 92, 93, 94, 95, 96, 97, 98, 99, 99.5, or 100% sequence identity to SEQ ID NO: 18. In an embodiment, the reverse adapter sequence with at least 85 %sequence identity has the same length as SEQ ID NO: 18.

Hence, the fusion primers may comprise from 20 to 100 nucleotides, preferably, 33 to 70 nucleotides; more preferably 45 to 60 nucleotides.

In one embodiment, the primer pair is a pair of specific primers without adapters. The specific sequence of the primer may comprise from 5 to 50 nucleotides, preferably, 8 to 30 nucleotides; more preferably 15 to 25 nucleotides. The specific sequences of the primer are complementary to at least a portion of the sequence to be reverse transcribed or amplified. Preferably, the specific sequences of primer s are fully complementary to a sequence within the sequence to be reverse transcribed or amplified.

The forward primer of the primer pair specific for attenuation locus 1 may have at least 85, 86, 87, 88, 89, 90, 91, 92, 93, 94, 95, 96, 97, 98, 99, 99.5, or 100% sequence identity to SEQ ID NO: 11. In an embodiment, the forward primer with at least 85% sequence identity has the same length as SEQ ID NO: 11.

The reverse primer of the primer pair specific for attenuation locus 1 may have at least 85, 86, 87, 88, 89, 90, 91, 92, 93, 94, 95, 96, 97, 98, 99, 99.5, or 100% sequence identity to SEQ ID NO: 12. In an embodiment, the reverse primer with at least 85% sequence identity has the same length as SEQ ID NO: 12.

The forward primer of the primer pair specific for attenuation locus 2 may have at least 85, 86, 87, 88, 89, 90, 91, 92, 93, 94, 95, 96, 97, 98, 99, 99.5, or 100% sequence identity to SEQ ID NO: 13. In an embodiment, the forward primer with at least 85% sequence identity has the same length as SEQ ID NO: 13.

The reverse primer of the primer pair specific for attenuation locus 2 may have at least 85, 86, 87, 88, 89, 90, 91, 92, 93, 94, 95, 96, 97, 98, 99, 99.5, or 100% sequence identity to SEQ ID NO: 14. In an embodiment, the reverse primer with at least 85% sequence identity has the same length as SEQ ID NO: 14.

The forward primer of the primer pair specific for attenuation locus 3 may have at least 85, 86, 87, 88, 89, 90, 91, 92, 93, 94, 95, 96, 97, 98, 99, 99.5, or 100% sequence identity to SEQ ID NO: 15. In an embodiment, the forward primer with at least 85% sequence identity has the same length as SEQ ID NO: 15.

The reverse primer of the primer pair specific for attenuation locus 3 may have at least 85, 86, 87, 88, 89, 90, 91, 92, 93, 94, 95, 96, 97, 98, 99, 99.5, or 100% sequence identity to SEQ ID NO: 16. In an embodiment, the reverse primer with at least 85% sequence identity has the same length as SEQ ID NO: 16.

The three attenuation loci referred to herein are termed attenuation locus 1, attenuation locus 2, and attenuation locus 3. Attenuation locus 1 comprises a C-to-T mutation at genomic nucleotide position 57 in the 5' noncoding region. This is also referred to as attenuated genotype 1. Attenuation locus 2 comprises a G-to-A mutation at genomic nucleotide position 2579 (causing a Gly-to-Asp mutation at amino acid position 53 in the nonstructural protein (NS) 1. This is also referred to as attenuated genotype 2. Attenuation locus 3 comprises an A-to-T mutation at genomic nucleotide position 5270 (causing a Glu-to-Val mutation at amino acid position 250 in the nonstructural protein (NS) 3. This is also referred to as attenuated genotype 3. These three genetic determinants of attenuation reside outside of the structural gene regions of the TDV-2 viral genome. The numbering follows the occurrence of the attenuation loci along the DEN-2 PDK-53 backbone. When one or more of the attenuation mutations is present in a genome, in an isolated polynucleotide or in an amplified polynucleotide, this may be referred to as an "attenuated genotype".

The specific sequences of the fusion primer pairs are specific for one of the three attenuation loci. Hence, the fusion primers are termed ATT1, ATT2 or ATT3 (for attenuation locus 1, 2 or 3) and F (for forward) or R (for reverse). Hence, ATT1 primers preferably bind in the 5' noncoding region, ATT2 primers preferably bind in the nonstructural protein (NS) 1 and ATT 3 primers preferably bind in the nonstructural protein (NS) 3. Hence, the fusion primers bind outside of the structural gene regions of the TDV viral genome.

Accordingly, in any of the methods disclosed herein, the amplicon generated by the ATT-1 specific fusion primer pair may have a length of about 324 bp, the amplicon generated by the ATT-2 specific fusion primer pair may have a length of about 333bp, the amplicon generated by the ATT-3 specific fusion primer pair may have a length of about 408 bp.

The amplicon comprises the sequence of interest comprising the specific primer sequence and the forward and reverse adapter sequences. In one embodiment, each fusion primer consists of a specific primer sequence and an adapter sequence. In one embodiment, the forward adapter sequence has a length of 33 bp and the reverse adapter sequence has a length of 34 bp. Hence, in one embodiment, the forward and reverse adapter sequences together have a length of 67 bp. In one embodiment, the specific primer sequences may be used as stand-alone primers without the adapter sequences. Hence, in one embodiment, the primers are not fusion primers. In this case, the amplicon generated by the specific primer sequences is 67 bp shorter than the amplicon generated by the fusion primers.

The term "genotype" as used herein refers to the presence of a specific nucleotide sequence in a genome. For example, the term "attenuation genotype" or "attenuated genotype" refers to the presence of the nucleotide sequence that has been identified as contributing to the attenuation phenotype. The attenuation genotype may be present in the RNA genome and/or in a DNA polynucleotide generated by reverse transcription of the RNA genome and/or in a DNA polynucleotide generated by PCR.

"Separating the generated amplicon(s)" herein means that the PCR product(s) obtained in step c) are separated by size. Any of the well-known methods in the art for separating DNA molecules by size may be used. In a preferred embodiment of any of the above methods, step d) thus includes gel electrophoresis. Gel electrophoresis is a laboratory method used to separate mixtures of DNA according to molecular size. In gel electrophoresis, the molecules to be separated are pushed by an electrical field through a gel that contains small pores. The molecules travel through the pores in the gel at a speed that is inversely related to their lengths. This means that a small DNA molecule will travel a greater distance through the gel than will a larger DNA molecule. The differently sized molecules will thus migrate in bands. The electrical field is applied such that one end of the gel has a positive charge and the other end has a negative charge. Because DNA consist of negatively charged molecules, these molecules will be pulled toward the positively charged end of the gel. Finally, after the DNA molecules have been separated using gel electrophoresis, bands representing molecules of different sizes can be detected. Preferably, the gel used for gel electrophoresis is an agarose gel. The percentage of agarose in the agarose gel preferably is 0.5-3%, more preferably 1-2%, most preferably 2%. In another preferred embodiment, the PCR product(s) obtained in step c) are separated by capillary electrophoresis. Capillary electrophoresis is well-known to the skilled person. Preferably, the capillary electrophoresis is a capillary gel electrophoresis, wherein the separation of the nucleic acids is performed in a capillary filled with a gel containing a dye. During the electric field-forced migration of the nucleic acids through the gel the dye attaches to the nucleic acids and is determined by a detector. Commercially available electrophoresis devices and cartridges include the QIAxcel® Advanced System and cartridges from Qiagen.

In an embodiment of any of the above inventive methods, the sample comprises residual host cell DNA. While host cell DNA may be removed during step a), this is not necessary for the inventive method. The specificity of the inventive primer pairs is such that no unspecific signal will be generated in step c) even in the presence of host cell DNA.

"Host cell" as used herein can mean cells used for the production of vaccine strains, preferably live, attenuated dengue virus strains, more preferably TDV-1 to TDV-4. Suitable host cells for vaccine strain production include mammalian cells such as, e.g., Vero cells. Accordingly, in a preferred embodiment of any of the above methods, prior to step a), the live, attenuated dengue virus is grown in Vero cells. It is preferred that Vero cells are seeded in T25 cm$^2$ flasks at $1.5 \times 10^6$ cells/flask and grown overnight to 80-90% confluency by incubating at 37°C with 5% $CO_2$ tension. Preferably, the virus is diluted in assay medium DMEM containing 1% FBS and 1% Penicillin/Streptomycin. Preferably, Vero cells are infected with TDV strains (e.g. TDV-1 to TDV-4) at 0.1MOI and allowed virus adsorption for 1 hour at 37°C 5% $CO_2$ incubator.

In a preferred embodiment the amplifying step c) results in a PCR product having a length of about 300 bp to 500 bp.

Steps a) to c) of the method can be referred to as "library preparation"

As used herein, "next generation sequencing (NGS) by synthesis" refers to a sequencing technology that can process multiple individual samples in parallel (multiplex) and creates short sequencing reads of up to about 390 bp length. NGS uses spatially separated, clonally amplified DNA templates (clusters) in a flow cell. Examples of NGS platforms are Roche 454, Illumina MiSeq, Illumina HiSeq, or Life Technologies SOLiD4. In one embodiment, the next generation sequencing by synthesis of the method comprises sequencing by reversible terminator chemistry. One example of reversible terminator chemistry sequencing is MiSeq sequencing.

Reversible terminator chemistry comprises nucleotide incorporation, fluorescence imaging and cleavage. Each individual amplicon template is anchored on a chip or flow cell as a single strand of DNA and clonally amplified, resulting in template strand clusters.

In a preferred embodiment before the sequencing step) bridge amplification PCR using oligonucleotides complementary to the 5' end of the indexed PCR product is carried out, wherein the oligonucleotides are immobilized on a support.

The principle of bridge amplification PCR is known to the skilled person. Oligonucleotides complementary to index sequence at the 5' end of the forward and reverse strand, respectively of a double-stranded DNA are immobilized on a support. When a single strand of the double-stranded DNA binds to the complementary oligonucleotide, the strand bends over and atttaches to a second oligonucleotide thereby forming a bridge. A primer then synthesizes the reverse strand. The two strands release and straighten. Each forms a new bridge (bridge amplification). The result is a cluster of DNA forward and reverse strand clones.

A fluorescently labelled reversible terminator is imaged as each dNTP is added, and then cleaved to allow incorporation of the next base. The emission wavelength and intensity are used to identify the base. The cycle is repeated n times to create a read length of n bases. MiSeq sequencing of PCR products is also referred to as amplicon sequencing.

In one embodiment of the step of next generation sequencing by synthesis, at least 12000 sequence reads, preferably at least 20000 sequence reads, more preferably at least 30000 sequence reads are analysed. In one embodiment, next generation sequencing by synthesis is reversible terminator chemistry sequencing and at least 12000 sequence reads, preferably at least 20000 sequence reads, more preferably at least 30000 sequence reads are analysed. A coverage of 12000 sequence may achieve a detection limit of 0.3% sequence variation.

In one embodiment, the ratio of the attenuated genotype of a live, attenuated flavivirus in the formulation is between about 80% and about 100%. Preferably, the ratio of the attenuated genotype of a live, attenuated flavivirus in the formulation is between about 90% and about 100%. More preferably, the ratio of the attenuated genotype of a live, attenuated flavivirus in the formulation is between about 95% and about 100%. Even more preferably, the ratio of the attenuated genotype of a live, attenuated flavivirus in the formulation is between about 99% and about 100%.

In one embodiment, the ratio of the attenuated genotype of a live, attenuated flavivirus in the formulation is above 90%. Preferably, the ratio of the attenuated genotype of a live, attenuated flavivirus in the formulation is above 95%. More preferably, the ratio of the attenuated genotype of a live, attenuated flavivirus in the formulation is above 99%.

The ratio of the number of sequence reads in which the nucleotide at the at least one attenuation locus is identical to the nucleotide of the live, attenuated flavivirus at the at least one attenuation locus to the total number of sequence reads quantifies the ratio of the attenuated genotype, i.e. the number of sequence reads in which at least one attenuated genotype is present divided by the total number of sequence reads.

In one embodiment, the attenuated genotype of the at least one live, attenuated flavivirus in the formulation is ATT1 and the ratio of the attenuated genotype of the at least one live, attenuated flavivirus in the formulation is above 90%.

In one embodiment, the attenuated genotype of the at least one live, attenuated flavivirus in the formulation is ATT1 and ATT2 and the ratio of the attenuated genotype of the at least one live, attenuated flavivirus in the formulation is above 90% for each of ATT1 and ATT2.

In one embodiment, the attenuated genotype of the at least one live, attenuated flavivirus in the formulation is ATT1 and ATT3 and the ratio of the attenuated genotype of the at least one live, attenuated flavivirus in the formulation is above 90% for each of ATT1 and ATT3.

In one embodiment, the attenuated genotype of the at least one live, attenuated flavivirus in the formulation is ATT2 and ATT3 and the ratio of the attenuated genotype of the at least one live, attenuated flavivirus in the formulation is above 90% for each of ATT2 and ATT3.

In one embodiment, the attenuated genotype of the at least one live, attenuated flavivirus in the formulation is ATT1, ATT2 and ATT3 and the ratio of the attenuated genotype of the at least one live, attenuated flavivirus in the formulation is above 90% for each of ATT1, ATT2 and ATT3. In one embodiment, the attenuated genotype of the at least one live, attenuated flavivirus in the formulation is ATT1, ATT2 and ATT3 and the ratio of the attenuated genotype of the at least one live, attenuated flavivirus in the formulation is above 99% for each of ATT1, ATT2 and ATT3.

In one embodiment, the at least one live, attenuated flavivirus is TDV-1 and the at least one attenuated genotype of TDV-1 in the formulation is ATT1 and the ratio of the attenuated genotype of TDV-1 in the formulation is above 90%.

In one embodiment, the at least one live, attenuated flavivirus is TDV-1 and the at least one attenuated genotype of TDV-1 in the formulation is ATT1 and ATT2 and the ratio of the attenuated genotype of TDV-1 in the formulation is above 90% for each of ATT1 and ATT2.

In one embodiment, the at least one live, attenuated flavivirus is TDV-1 and the at least one attenuated genotype of TDV-1 in the formulation is ATT1 and ATT3 and the ratio of the attenuated genotype of TDV-1 in the formulation is above 90% for each of ATT1 and ATT3.

In one embodiment, the at least one live, attenuated flavivirus is TDV-1 and the at least one attenuated genotype of TDV-1 in the formulation is ATT2 and ATT3 and the ratio of the attenuated genotype of TDV-1 in the formulation is above 90% for each of ATT2 and ATT3.

In one embodiment, the at least one live, attenuated flavivirus is TDV-1 and the at least one attenuated genotype of TDV-1 in the formulation is ATT1, ATT2 and ATT3 and the ratio of the attenuated genotype of TDV-1 in the formulation is above 90% for each of ATT1, ATT2 and ATT3. In one embodiment, the at least one live, attenuated flavivirus is TDV-1 and the at least one attenuated genotype of TDV-1 in the formulation is ATT1, ATT2 and ATT3 and the ratio of the attenuated genotype of TDV-1 in the formulation is above 99% for each of ATT1, ATT2 and ATT3.

In one embodiment, the at least one live, attenuated flavivirus is TDV-2 and the at least one attenuated genotype of TDV-2 in the formulation is ATT1 and the ratio of the attenuated genotype of TDV-2 in the formulation is above 90%.

In one embodiment, the at least one live, attenuated flavivirus is TDV-2 and the at least one attenuated genotype of TDV-2 in the formulation is ATT1 and ATT2 and the ratio of the attenuated genotype of TDV-2 in the formulation is above 90% for each of ATT1 and ATT2.

In one embodiment, the at least one live, attenuated flavivirus is TDV-2 and the at least one attenuated genotype of TDV-2 in the formulation is ATT1 and ATT3 and the ratio of the attenuated genotype of TDV-2 in the formulation is above 90% for each of ATT1 and ATT3.

In one embodiment, the at least one live, attenuated flavivirus is TDV-2 and the at least one attenuated genotype of TDV-2 in the formulation is ATT2 and ATT3 and the ratio of the attenuated genotype of TDV-2 in the formulation is above 90% for each of ATT2 and ATT3.

In one embodiment, the at least one live, attenuated flavivirus is TDV-2 and the at least one attenuated genotype of TDV-2 in the formulation is ATT1, ATT2 and ATT3 and the ratio of the attenuated genotype of TDV-2 in the formulation is above 90% for each of ATT1, ATT2 and ATT3. In one embodiment, the at least one live, attenuated flavivirus is TDV-2 and the at least one attenuated genotype of TDV-2 in the formulation is ATT1, ATT2 and ATT3 and the ratio of the attenuated genotype of TDV-2 in the formulation is above 99% for each of ATT1, ATT2 and ATT3.

In one embodiment, the at least one live, attenuated flavivirus is TDV-3 and the at least one attenuated genotype of TDV-3 in the formulation is ATT1 and the ratio of the attenuated genotype of TDV-3 in the formulation is above 90%.

In one embodiment, the at least one live, attenuated flavivirus is TDV-3 and the at least one attenuated genotype of TDV-3 in the formulation is ATT1 and ATT2 and the ratio of the attenuated genotype of TDV-3 in the formulation is above 90% for each of ATT1 and ATT2.

In one embodiment, the at least one live, attenuated flavivirus is TDV-3 and the at least one attenuated genotype of TDV-3 in the formulation is ATT1 and ATT3 and the ratio of the attenuated genotype of TDV-3 in the formulation is above 90% for each of ATT1 and ATT3.

In one embodiment, the at least one live, attenuated flavivirus is TDV-3 and the at least one attenuated genotype of TDV-3 in the formulation is ATT2 and ATT3 and the ratio of the attenuated genotype of TDV-3 in the formulation is above 90% for each of ATT2 and ATT3.

In one embodiment, the at least one live, attenuated flavivirus is TDV-3 and the at least one attenuated genotype of TDV-3 in the formulation is ATT1, ATT2 and ATT3 and the ratio of the attenuated genotype of TDV-3 in the formulation is above 90% for each of ATT1, ATT2 and ATT3. In one embodiment, the at least one live, attenuated flavivirus is TDV-3 and the at least one attenuated genotype of TDV-3 in the formulation is ATT1, ATT2 and ATT3 and the ratio of the attenuated genotype of TDV-3 in the formulation is above 99% for each of ATT1, ATT2 and ATT3.

In one embodiment, the at least one live, attenuated flavivirus is TDV-4 and the at least one attenuated genotype of TDV-4 in the formulation is ATT1 and the ratio of the attenuated genotype of TDV-4 in the formulation is above 90%.

In one embodiment, the at least one live, attenuated flavivirus is TDV-4 and the at least one attenuated genotype of TDV-4 in the formulation is ATT1 and ATT2 and the ratio of the attenuated genotype of TDV-4 in the formulation is above 90% for each of ATT1 and ATT2.

In one embodiment, the at least one live, attenuated flavivirus is TDV-4 and the at least one attenuated genotype of TDV-4 in the formulation is ATT1 and ATT3 and the ratio of the attenuated genotype of TDV-4 in the formulation is above 90% for each of ATT1 and ATT3.

In one embodiment, the at least one live, attenuated flavivirus is TDV-4 and the at least one attenuated genotype of TDV-4 in the formulation is ATT2 and ATT3 and the ratio of the attenuated genotype of TDV-4 in the formulation is above 90% for each of ATT2 and ATT3.

In one embodiment, the at least one live, attenuated flavivirus is TDV-4 and the at least one attenuated genotype of TDV-4 in the formulation is ATT1, ATT2 and ATT3 and the ratio of the attenuated genotype of TDV-4 in the formulation is above 90% for each of ATT1, ATT2 and ATT3. In one embodiment, the at least one live, attenuated flavivirus is TDV-4 and the at least one attenuated genotype of TDV-4 in the formulation is ATT1, ATT2 and ATT3 and the ratio of the attenuated genotype of TDV-4 in the formulation is above 99% for each of ATT1, ATT2 and ATT3.

In one embodiment, the at least one live, attenuated flavivirus is TDV-1 and TDV-2 and the at least one attenuated genotype of TDV-1 and TDV-2 in the formulation is ATT1 and the ratio of the attenuated genotype of TDV-1 and TDV-2 in the formulation is above 90%.

In one embodiment, the at least one live, attenuated flavivirus is TDV-1 and TDV-2 and the at least one attenuated genotype of TDV-1 and TDV-2 in the formulation is ATT1 and ATT2 and the ratio of the attenuated genotype of TDV-1 and TDV-2 in the formulation is above 90% for each of ATT1 and ATT2.

In one embodiment, the at least one live, attenuated flavivirus is TDV-1 and TDV-2 and the at least one attenuated genotype of TDV-1 and TDV-2 in the formulation is ATT1 and ATT3 and the ratio of the attenuated genotype of TDV-1 and TDV-2 in the formulation is above 90% for each of ATT1 and ATT3.

In one embodiment, the at least one live, attenuated flavivirus is TDV-1 and TDV-2 and the at least one attenuated genotype of TDV-1 and TDV-2 in the formulation is ATT2 and ATT3 and the ratio of the attenuated genotype of TDV-1 and TDV-2 in the formulation is above 90% for each of ATT2 and ATT3.

In one embodiment, the at least one live, attenuated flavivirus is TDV-1 and TDV-2 and the at least one attenuated genotype of TDV-1 and TDV-2 in the formulation is ATT1, ATT2 and ATT3 and the ratio of the attenuated genotype of TDV-1 and TDV-2 in the formulation is above 90% for each of ATT1, ATT2 and ATT3. In one embodiment, the at least one live, attenuated flavivirus is TDV-1 and TDV-2 and the at least one attenuated genotype of TDV-1 and TDV-2 in the formulation is ATT1, ATT2 and ATT3 and the ratio of the attenuated genotype of TDV-1 and TDV-2 in the formulation is above 99% for each of ATT1, ATT2 and ATT3.

In one embodiment, the at least one live, attenuated flavivirus is TDV-1, TDV-2 and TDV-3 and the at least one attenuated genotype of TDV-1, TDV-2 and TDV-3 in the formulation is ATT1 and the ratio of the attenuated genotype of TDV-1, TDV-2 and TDV-3 in the formulation is above 90%.

In one embodiment, the at least one live, attenuated flavivirus is TDV-1, TDV-2 and TDV-3 and the at least one attenuated genotype of TDV-1, TDV-2 and TDV-3 in the formulation is ATT1 and ATT2 and the ratio of the attenuated genotype of TDV-1, TDV-2 and TDV-3 in the formulation is above 90% for each of ATT1 and ATT2.

In one embodiment, the at least one live, attenuated flavivirus is TDV-1, TDV-2 and TDV-3 and the at least one attenuated genotype of TDV-1, TDV-2 and TDV-3 in the formulation is ATT1 and ATT3 and the ratio of the attenuated genotype of TDV-1, TDV-2 and TDV-3 in the formulation is above 90% for each of ATT1 and ATT3.

In one embodiment, the at least one live, attenuated flavivirus is TDV-1, TDV-2 and TDV-3 and the at least one attenuated genotype of TDV-1, TDV-2 and TDV-3 in the formulation is ATT2 and ATT3 and the ratio of the attenuated genotype of TDV-1, TDV-2 and TDV-3 in the formulation is above 90% for each of ATT2 and ATT3.

In one embodiment, the at least one live, attenuated flavivirus is TDV-1, TDV-2 and TDV-3 and the at least one attenuated genotype of TDV-1, TDV-2 and TDV-3 in the formulation is ATT1, ATT2 and ATT3 and the ratio of the attenuated genotype of TDV-1, TDV-2 and TDV-3 in the formulation is above 90% for each of ATT1, ATT2 and ATT3. In one embodiment, the at least one live, attenuated flavivirus is TDV-1, TDV-2 and TDV-3 and the at least one attenuated genotype of TDV-1, TDV-2 and TDV-3 in the formulation is ATT1, ATT2 and ATT3 and the ratio of the attenuated genotype of TDV-1, TDV-2 and TDV-3 in the formulation is above 99% for each of ATT1, ATT2 and ATT3.

In one embodiment, the at least one live, attenuated flavivirus is TDV-1, TDV-2 , TDV-3 and TDV-4 and the at least one attenuated genotype of TDV-1, TDV-2 , TDV-3 and TDV-4 in the formulation is ATT1 and the ratio of the attenuated genotype of TDV-1, TDV-2 , TDV-3 and TDV-4 in the formulation is above 90%.

In one embodiment, the at least one live, attenuated flavivirus is TDV-1, TDV-2 , TDV-3 and TDV-4 and the at least one attenuated genotype of TDV-1, TDV-2 , TDV-3 and TDV-4 in the formulation is ATT1 and ATT2 and the ratio of the attenuated genotype of TDV-1, TDV-2 , TDV-3 and TDV-4 in the formulation is above 90% for each of ATT1 and ATT2.

In one embodiment, the at least one live, attenuated flavivirus is TDV-1, TDV-2 , TDV-3 and TDV-4 and the at least one attenuated genotype of TDV-1, TDV-2 , TDV-3 and TDV-4 in the formulation is ATT1 and ATT3 and the ratio of the attenuated genotype of TDV-1, TDV-2 , TDV-3 and TDV-4 in the formulation is above 90% for each of ATT1 and ATT3.

In one embodiment, the at least one live, attenuated flavivirus is TDV-1, TDV-2 , TDV-3 and TDV-4 and the at least one attenuated genotype of TDV-1, TDV-2 , TDV-3 and TDV-4 in the formulation is ATT2 and ATT3 and the ratio of the attenuated genotype of TDV-1, TDV-2 , TDV-3 and TDV-4 in the formulation is above 90% for each of ATT2 and ATT3.

In one embodiment, the at least one live, attenuated flavivirus is TDV-1, TDV-2 , TDV-3 and TDV-4 and the at least one attenuated genotype of TDV-1, TDV-2 , TDV-3 and TDV-4 in the formulation is ATT1, ATT2 and ATT3 and the ratio of the attenuated genotype of TDV-1, TDV-2 , TDV-3 and TDV-4 in the formulation is above 90% for each of ATT1, ATT2 and ATT3. In one embodiment, the at least one live, attenuated flavivirus is TDV-1, TDV-2 , TDV-3 and TDV-4 and the at least one attenuated genotype of TDV-1, TDV-2 , TDV-3 and TDV-4 in the formulation is ATT1, ATT2 and ATT3 and the ratio of the attenuated genotype of TDV-1, TDV-2, TDV-3 and TDV-4 in the formulation is above 99% for each of ATT1, ATT2 and ATT3.

Where the ratio of the attenuated genotype of more than one dengue serotype in the formulation is determined, the sum of the attenuated genotype of the more than one dengue serotypes is used. Thus, the ratio of the attenuated genotype means the sum of sequencing reads with at least one attenuated genotype from all dengue serotypes divided by the total sequencing reads.

In another embodiment a method is provided wherein the live, attenuated flavivirus is a dengue virus comprising three attenuation loci at positions 57, 2579 and 5270 of the nucleotide sequence of dengue virus type 2 according to SEQ ID NO: 2 and the primer pair of step c) is selected from the group consisting of:

i) a forward primer comprising SEQ ID NO: 11, or a variant thereof having at least 85% sequence identity thereof and a reverse primer comprising SEQ ID NO: 12, or a variant thereof having at least 85% sequence identity thereof such that a first PCR product having a length of about 310 bp to about 330 bp and comprising the first attenuation locus is generated;

ii) a forward primer comprising SEQ ID NO: 13, or a variant thereof having at least 85% sequence identity thereof and a reverse primer pair comprising SEQ ID NO: 14, or a variant thereof having at least 85% sequence identity thereof such that a second PCR product having a length of about 320 bp to about 350 bp and comprising the second attenuation locus is generated; and

iii) a forward primer comprising SEQ ID NO: 15, or a variant thereof having at least 85% sequence identity thereof and a reverse primer comprising SEQ ID NO: 16, or a variant thereof having at least 85% sequence identity thereof such that a third PCR product having a length of about 400 bp to about 430 bp and comprising the third attenuation locus is generated.

In another aspect a method is provided wherein the live, attenuated flavivirus is a dengue virus comprising three attenuation loci at positions 57, 2579 and 5270 of the nucleotide sequence of dengue virus type 2 according to SEQ ID NO: 2 and the primer pair of step c) is selected from the group consisting of:

i) a forward primer comprising SEQ ID NO: 5, or a variant thereof having at least 85% sequence identity thereof and a reverse primer comprising SEQ ID NO: 6, or a variant thereof having at least 85% sequence identity thereof such that a first PCR product having a length of about 310 bp to about 330 bp and comprising the first attenuation locus is generated;

ii) a forward primer comprising SEQ ID NO: 7, or a variant thereof having at least 85% sequence identity thereof and a reverse primer pair comprising SEQ ID NO: 8, or a variant thereof having at least 85% sequence identity thereof such that a second PCR product having a length of about 320 bp to about 350 bp and comprising the second attenuation locus is generated; and

iii) a forward primer comprising SEQ ID NO: 9, or a variant thereof having at least 85% sequence identity thereof and a reverse primer comprising SEQ ID NO: 10, or a variant thereof having at least 85% sequence identity thereof such that a third PCR product having a length of about 400 bp to about 430 bp and comprising the third attenuation locus is generated.

In one embodiment, the first PCR product comprising the first attenuation locus has a length of 324 bp, the second PCR product comprising the second attenuation locus has a length of 333 bp and the third PCR product comprising the third attenuation locus has a length of 408 bp

In another embodiment of the method after step c) one or more of the following steps is/are performed:

(i) purifying the PCR products;
(ii) performing an Index-PCR reaction on the PCR products of step c) using forward and reverse index primer pairs thereby obtaining indexed PCR products; and/or
(iii) purifying the PCR products of step (ii).

As used herein, "purifying the PCR products" refers to a process of removing reagents and/or buffers from a mixture comprising the PCR product. After PCR, the mixture comprises the PCR products (amplicons), dNTPs, oligonucleotides, enzymes, e.g. polymerase, and template. The amplicons are separated from the mixture using, for example, a DNA binding column or magnetic beads. Suitable methods comprise inter alia steps such as centrifugation steps, precipitation steps, chromatography steps, dialyzing steps, heating steps, cooling steps and/or denaturation steps. Amplicons may be eluted into a new diluent, for example buffer or water. Any commercially available kit for the purification of nucleic acids may be used.

In order to prepare PCR products for sequencing, an indexed library may be prepared. Hence, the method may comprise the step of performing an Index-PCR reaction on the PCR products of step c) using forward and reverse index primer pairs thereby obtaining indexed PCR products. The term "index primers" refers to primers used for indexed library preparation. They are used in an "index PCR" reaction whereby indexes are attached to the amplicon comprising adapter sequences and sequence of interest. The sequence of interest is an attenuation locus. Consequently, the amplicon can be referred to as an "indexed amplicon". Hence, each amplicon can be identified by a unique tag or index. After index PCR, each amplicon comprises the sequence of interest, adapter sequences and indexes. For index PCR, Nextera XT Index Kit may be used (Illumina, catalog number FC-131-1002)

Hence, after PCR with the fusion primers comprising specific sequences and adapter sequences (step c) ), an index PCR is performed, whereby index primers bind to the adapter sequences. The reaction may be carried out in an automated thermal cycler.

In one embodiment, the indexed amplicons of the at least one attenuation locus are 69 bp longer than the amplicon from step c).

Thus, one embodiment of the invention as defined by the appended claims 1-14 relates to a method for determining the proportion of an attenuated genotype of a live, attenuated flavivirus in a formulation

According to a second aspect, the present invention as defined by the appended claims provides the use of any of the inventive methods according to the first aspect of the invention in the quality control of vaccines containing at least one live, attenuated flavivirus. wherein the at least one live, attenuated flavivirus is a dengue virus. For the reliable determination of the serotypes of both the monovalent drug substance comprising a single live, attenuated dengue virus and the tetravalent drug product comprising a mixture of all four live, attenuated viruses it is essential to confirm the presence of (only) the dengue virus serotype(s) in the vaccine formulation before batches are released for administration to individuals.

The inventive method as defined by the appended claims for the quality control of vaccines comprising at least one live, attenuated flavivirus comprising comprises performing the method of the first aspect of the invention and performing a quality control step, wherein the vaccine is accepted if the proportion of the at least one live, attenuated virus in the vaccine is at least 90%, preferably at least 95 %, more preferably at least 97% and most preferably at least 99%.

According to a third aspect of the present invention, as defined by the appended claims a quality control method for vaccines containing live, attenuated dengue virus is provided comprising or consisting of performing the method of the first aspect of the invention and at least one further method selected from the group consisting of immunofocus assay, visual inspection, determination of reconstitution time or resuspendability of lyophilisates, sterility test, test for bacterial endotoxins, determination of host cell DNA, determination of pH, colorimetric determination of water, determination of osmolality, determination of the content of one or more excipient(s). In an embodiment, the quality control method comprises or consists of the method of the first aspect of the invention and at least two further method selected from the group consisting of immunofocus assay, visual inspection, determination of reconstitution time or resuspendability of lyophilisates, sterility test, test for bacterial endotoxins, determination of host cell DNA, determination of pH, colorimetric determination of water, determination of osmolality, determination of the content of one or more excipient(s). In an embodiment, the quality control method comprises or consists of the method of the first aspect of the invention and at least three further method selected from the group consisting of immunofocus assay, visual inspection, determination of reconstitution time or resuspendability of lyophilisates, sterility test, test for bacterial endotoxins, determination of host cell DNA, determination of pH, colorimetric determination of water, determination of osmolality, determination of the content of one or more excipient(s). In an embodiment, the quality control method comprises or consists of the method of the first aspect of the invention and at least four further method selected from the group consisting of immunofocus assay, visual inspection, determination of reconstitution time or resuspendability of lyophilisates, sterility test, test for bacterial endotoxins, determination of host cell DNA, determination of pH, colorimetric determination of water, determination of osmolality, determination of the content of one or more excipient(s). In an embodiment, the quality control method comprises or consists of the method of the first aspect of the invention and at least five further method selected from the group consisting of immunofocus assay, visual inspection, determination of reconstitution time or resuspendability of lyophilisates, sterility test, test for bacterial endotoxins, determination of host cell DNA, determination of pH, colorimetric determination of water, determination of osmolality, determination of the content of one or more excipient(s). In an embodiment, the quality control method comprises or consists of the method of the first aspect of the invention and at least six further method selected from the group consisting of immunofocus assay, visual inspection, determination of reconstitution time or resuspendability of lyophilisates, sterility test, test for bacterial endotoxins, determination of host cell DNA, determination of pH, colorimetric determination of water, determination of osmolality, determination of the content of one or more excipient(s). In an embodiment, the quality control method comprises or consists of the method of the first aspect of the invention and at least seven further method selected from the group consisting of immunofocus assay, visual inspection, determination of reconstitution time or resuspendability of lyophilisates, sterility test, test for bacterial endotoxins, determination of host cell DNA, determination of pH, colorimetric determination of water, determination of osmolality, determination of the content of one or more excipient(s). In an embodiment, the quality control method comprises or consists of the method of the first aspect of the invention and at least eight further method selected from the group consisting of immunofocus assay, visual inspection, determination of reconstitution time or resuspendability of lyophilisates, sterility test, test for bacterial endotoxins, determination of host cell DNA, determination of pH, colorimetric determination of water, determination of osmolality, determination of the content of one or more excipient(s). In an embodiment, the quality control method comprises or consists of the method of the first aspect of the invention and at least nine further method selected from the group consisting of immunofocus assay, visual inspection, determination of reconstitution time or resuspendability of lyophilisates, sterility test, test for bacterial endotoxins, determination of host cell DNA, determination of pH, colorimetric determination of water, determination of osmolality, determination of the content of one or more excipient(s). In an embodiment, the quality control method comprises or consists of the method of the first aspect of the invention and all of immunofocus assay, visual inspection, determination of reconstitution time or resuspendability of lyophilisates, sterility test, test for bacterial endotoxins, determination of host cell DNA, determination of pH, colorimetric determination of water, determination of osmolality, determination of the content of one or more excipient(s).

As used herein, the term "excipient" refers to a substance added to a liquid pharmaceutical composition in addition to the biological active agent. This can include substances used for the purpose of enhancing stabilization of the active agent, salts, carbohydrates (such as e.g., sugars), surfactants, proteins, bulking agents, fillers, or agents that in combination with the active agent can confer a therapeutic enhancement of the composition. In particular, excipients may refer to salts, carbohydrates, non-ionic surfactants and albumins. Excipients may also refer to buffers such as e.g., phosphate buffer. Excipients may also refer to buffers such as e.g. phosphate buffer. Phosphate buffer is obtainable by admixing potassium buffer salts such as e.g. potassium dihydrogen phosphate and disodium hydrogen phosphate, in a set ratio. Within the meaning of the disclosure phosphate buffer is considered as a single excipient. The excipients may be described by referring to the admixed (dry) excipients.

In one embodiment, the one or more excipients may be selected from the group consisting of trehalose, poloxamer, urea, arginine hydrochloride, tromethamine, human serum albumin, chloride salts, and phosphate salts or any combination thereof. Preferably, trehalose is $\alpha,\alpha$-trehalose dihydrate. Preferably, poloxamer is poloxamer 407, also known by its trade names Pluronic F127 and Synperonic PE/F127. Preferably, the chloride salts may comprise or consist of one or both of potassium chloride and sodium chloride. Preferably, the phosphate salts may comprise or consist of one or both of potassium dihydrogen phosphate and disodium hydrogen phosphate.

In one preferred such embodiment, the excipients are a combination of trehalose, at least one poloxamer, and human serum albumin. More preferably, the excipients are a combination of trehalose, poloxamer, human serum albumin, chloride salts, and phosphate salts. Most preferably, the excipients are a combination of $\alpha,\alpha$-trehalose dihydrate, poloxamer 407, human serum albumin, potassium chloride, sodium chloride, potassium dihydrogen phosphate, and disodium hydrogen phosphate.

In another preferred such embodiment, the excipients are a combination of trehalose, at least one poloxamer, urea, arginine hydrochloride, tromethamine and human serum albumin. More preferably, the excipients are a combination of trehalose, poloxamer, urea, arginine hydrochloride, tromethamine, human serum albumin, chloride salts and phosphate salts. Preferably, the chloride salts comprise or consist of sodium chloride.

The invention as defined by the appended claims is further described in the following examples which are solely for the purpose of illustrating specific embodiments

## Examples

### Abbreviations

| A | Adenine | PCR | Polymerase Chain Reaction |
|---|---|---|---|
| AC | Amplification Control | | Test Method |
| ATT | ATTenuation | PM PSC | Product Sequencing Control |
| bp | Base pair | R | Reverse |
| C | Cytosine | RNA | RiboNucleic Acid |
| DEN-2 | DENgue virus vaccine candidate | RT-PCR | Reverse Transcriptase PCR |
| DNA | DeoxyriboNucleic Acid | s | Second / Seconds |
| EC | Extraction Control | SC-AMPX | Sequencing Control for Amplicon sequencing with MiSeq |
| F | Forward | | generated from PCV2 and Nextera XT adapter |
| G | Guanine | SNP | Single Nucleotide Polymorphism |
| $H_2O_{Mol}$ | Water molecular biological grade | SOP | Standard Operation Procedure |
| IDT | IDT Biologika GmbH | SST | System or Sample Suitability Test |
| kb | Kilo base pairs | T | Thymine |
| M | Molar ( $= {}^{mol}/_l$ ) | TDV | Tetravalent Dengue Vaccine candidate |
| min | Minute / Minutes | T-QC-E3 | Excellence Team of Molecular biological quality control |
| NEC | Negative Extraction Control | WI | Working Instruction |
| nt | Nucleotides | $\times$ g | Multiple of gravitational constant |
| NTC | No Template Control | | |

### Materials

**Table 1** Samples

| Sample | Characteristics | Designation | Concentration |
|---|---|---|---|
| TDV-1 $1.32\times10^9\ {}^{FFU}/_{ml}$ | DEN-2 with enveloped protein sequence of serotype 1, purified virions | TDV1 BDS after filtration; TDV1-001-01-15-DS; SAP #1502090089 (0389595) | |
| TDV-2 $3.31\times10^8\ {}^{FFU}/_{ml}$ | DEN-2 with enveloped protein sequence of serotype 2, purified virions | TDV2 BDS after filtration; TDV2-001-10-14-DS; SAP #1410240147 (0389602) | |

(continued)

| Sample | Characteristics | Designation | Concentration |
|---|---|---|---|
| TDV-3 $5.62 \times 10^8 \, \text{FFU}/\text{ml}$ | DEN-2 with enveloped protein sequence of serotype 3, purified virions | TDV3 BDS after filtration; TDV3-001-11-14-DS; SAP #1412090100 (0389605) | |
| TDV-4 $3.02 \times 10^7 \, \text{FFU}/\text{ml}$ | DEN-2 with enveloped protein sequence of serotype 4, purified virions | TDV4 BDS after filtration; TDV4-001-11-14-DS; SAP #1411190023 (0389606) | |
| EC | Mixture of TDV-1, -2 and -3 spiked with 5% TDV-4 | see sensitivity assay of Table 17 | |
| NEC | Negative control ($H_2O_{Mol}$) which is treated in the same way like the samples | | |
| NTC | Negative control ($H_2O_{Mol}$) in the RT-PCR | | |

**Table 2** Reagents

| Reagent | Vendor | Catalog Number |
|---|---|---|
| Specific primers with sequencing adapter | Ella Biotech | Individual order |
| $H_2O_{Mol}$ | Lonza | BE51200 |
| Nuclease micrococcal from Staphylococcus aureus | Sigma-Aldrich | N3755-500UN |
| Calcium chloride solution BioUltra 1M | Sigma-Aldrich | 21115-100ML |
| 0.5 M EDTA pH 8.0 | Lonza | 51201 |
| High Pure Viral Nucleic Acid Kit | Roche Diagnostics | 11858874001 |
| Titan One Tube RT-PCR System | Roche Diagnostics | 11939823001 |
| FlashGel Loading Dye 5X Concentration | Lonza | 50463 |
| FlashGel DNA Cassette 1.2% Agarose | Lonza | 57023 |
| FlashGel DNA Marker 100-3000bp | Lonza | 57034 |
| AMPure XP Beads | Beckman Coulter | A63880, A63881 |
| Absolute Ethanol | AppliChem | A3678,1000 |
| 10mM Tris-HCl pH 8.5 mit 0.1% Tween 20 | Teknova | T7724 |
| Nextera XT Index Kit (96 Indexes, 384 Samples) | Illumina | FC-131-1002 |
| Phusion Flash High-Fidelity PCR Master Mix | Thermo Scientific | F-548L |
| Quant-iT PicoGreen dsDNA Assay Kit | Life technologies | P11496 |
| TE-Puffer | Lonza | 51235 |
| NaOH (10 N) | Sigma Aldrich | 72068-100ML |
| PhiX Control v3 | Illumina | FC-110-3001 |
| MiSeq Reagent Kit v3, 600 Cycles | Illumina | MS-102-3003 |
| Tween 20 | AppliChem | A4974,0100 |
| deionised water (18 M$\Omega$, 0.05 $\mu$S) | IDT | |

**Table 3** Equipment

| Equipment | Manufacturer |
|---|---|
| Calibrated Single channel pipette | Eppendorf |
| Calibrated Multichannel pipette | Eppendorf |
| PCR-workstation | Erlab Captair Bio |

(continued)

| Equipment | Manufacturer |
|---|---|
| Laminar Flow, Safety class 2 | Kendro, Steag, Thermo |
| Centrifuge | Eppendorf, PeqLab, Hermle |
| Vortexer | IKA Werke/MS 1 |
| Thermomixer | Eppendorf |
| PCR Cycler Veriti | Applied Biosystems |
| Power supply | Apelex, Lonza |
| Gel documentation system | Biostep, Vilber Lourmat |
| DynaMag | Life Technologies |
| Multimode reader | Tecan |
| MiSeq | Illumina |
| Refrigerator | Liebherr |
| -20°C Freezer | Liebherr |
| -80°C Freezer | Sanyo |

**Methods**

**Primer design**

**Table 4** Specific (s) primers sequences for the 3 attenuation sites for all 4 TDV serotypes

| Primer | Specific binding sequence | SEQ ID No. |
|---|---|---|
| ATT1_F s | 5'-CTACGTGGACCGACAAAGAC-3' | 11 |
| ATT2_F s | 5'-GAACTGAAATGTGGCAGTGGG-3' | 13 |
| ATT3_F s | 5'-AGCGGGAAAGACGAAGAGATAC-3' | 15 |
| ATT1_R s | 5'-TAGGAAACGAAGGAACGCCA-3' | 12 |
| ATT2_R s | 5'-CCTGCCTGCATGATTCCTTTG-3' | 14 |
| ATT3_R s | 5'-CTGCCTCACCCATCTCCACTC-3' | 16 |

For sequencing by the used technology the specific primers are fused with the Nextera XT overhanging adapter sequence. (Table 5)

**Table 5** Illumina overhang adapter for all primers

| Primer | Nextera XT Fusion primer: overhanging adapter sequence | SEQ ID No. |
|---|---|---|
| Forward | 5'-tcgtcggcagcgtcagatgtgtataagagacag-3' | 17 |
| Reverse | 5'-gtctcgtgggctcggagatgtgtataagagacag-3' | 18 |

**Table 6** Fusion primer to amplify and to sequence the 4 serotypes of TDV and TDV sequencing control

| Primer | Fusion Primer: overhanging adapter sequence + Specific binding sequence | SEQ ID No. |
|---|---|---|
| ATT1_F | 5'-tcgtcggcagcgtcagatgtgtataagagacagCTACGTGGACCGACAAAGAC-3' | 5 |
| ATT2_F | 5'-tcgtcggcagcgtcagatgtgtataagagacagGAACTGAAATGTGGCAGTGGG-3' | 7 |
| ATT3_F | 5'-tcgtcggcagcgtcagatgtgtataagagacagAGCGGGAAAGACGAAGAGATAC-3' | 9 |

(continued)

| Primer | Fusion Primer: overhanging adapter sequence + Specific binding sequence | SEQ ID No. |
|--------|---------------------------------------------------------------------------|------------|
| ATT1_R | 5'-gtctcgtgggctcggagatgtgtataagagacagTAGGAAACGAAGGAACGCCA-3' | 6 |
| ATT2_R | 5'-gtctcgtgggctcggagatgtgtataagagacagCCTGCCTGCATGATTCCTTTG-3' | 8 |
| ATT3_R | 5'-gtctcgtgggctcggagatgtgtataagagacagCTGCCTCACCCATCTCCACTC-3' | 10 |

## RNA Extraction

The extraction of target RNA was performed in two steps. At first the residual DNA was digested with Nuclease micrococcal (Table 7). Then RNA is purified using RNA extraction with High Pure Viral Nucleic Acid Kit (Table 8).

Poly (A) Carrier RNA, Proteinase K, Inhibitor Removal Buffer, and Wash Buffer were prepared according to the supplier information.

**Table 7** Digestion of non-enveloped nucleic acids

| |
|---|
| 165 $\mu$l Sample<br>+ 16.5 $\mu$l Nuclease micrococcal (0.2 U/$\mu$l)<br>+ 1.65 $\mu$l 0.1 M CaCl$_2$-Solution |
| Vortex & centrifuge briefly & incubate at 28°C, 30 min & 700 rpm at Thermomixer |
| Stop the reaction with 16.5 $\mu$l 0.5 M EDTA; storage at -80 °C for up to 2 weeks |

**Table 8** RNA extraction with High Pure Viral Nucleic Acid Kit

| |
|---|
| For each sample: add 4 $\mu$l Poly (A) Carrier RNA stock solution to 200 $\mu$l Binding Buffer & mix it carefully with the pipette (do not vortex!) = Lysis Buffer |
| Add 50 $\mu$l Proteinase K & 200 $\mu$l Lysis Buffer to the sample (200 $\mu$l), vortex 15 s |
| Incubate at 72°C, 10 min & 300 rpm at a Thermomixer |
| Add 100 $\mu$l Binding Buffer, vortex & centrifuge briefly |
| Transfer the sample on a High Pure Spin Filter Tube & centrifuge 1 min at 8 000 $\times$ g |
| Discard collection tube & place the filter tube on a new collection tube |
| Add 500 $\mu$l Inhibitor Removal Buffer & centrifuge 1 min at 8 000 $\times$ g |
| Discard collection tube & place the filter tube on a new collection tube |
| 2 $\times$ Add 450 $\mu$l Wash Buffer, centrifuge 1 min at 8 000 $\times$ g, Discard collection tube & place the filter tube on a new collection tube |
| Centrifuge 10 s at 13 000$\times$g |
| Discard collection tube & place the filter tube on a new 1.5 ml-Tube |
| Add 50 $\mu$l H$_2$O$_{Mol}$, incubate 1 min at room temperature, centrifuge 1 min at 8 000$\times$g |
| Discard filter tube & aliquot á 25 $\mu$l |

## RT-PCR

All primers were purchased from Ella Biotech as 100 $\mu$M stock solution. The primer stock solutions were diluted 1:20 and each forward and reverse primer were diluted to 5 $\mu$M each. The working solution can be stored up to 5 years at -20 °C. The reaction was prepared according to Table 9 and the PCR was performed according to Table 10.

**Table 9** The reaction mixture contains the following components

| Component | Concentration | e.g. for 1$\times$ |
|-----------|---------------|--------------------|
| H$_2$O$_{Mol}$ (Vial 6) | Residual volume | 12.625 $\mu$l |

(continued)

| Component | Concentration | e.g. for 1× |
|---|---|---|
| 5× RT-PCR buffer | onefold | 5.0 μl |
| dNTP mix, 10 mM (Vial 2) | 0.8 mM | 2.0 μl |
| DTT solution, 100 mM | 5 mM | 1.25 μl |
| Enzyme mix (Vial 1) | $1\ ^{\mu l}/_{50\mu l}$ | 0.5 μl |
| RNase Inhibitor, 5 U/μl (Vial 3) | 0.5 U | 0.125 μl |
| Forward+Reverse -Primer, 5 μM each | 0.2 μM | 1.0 μl |
| Sample | 2.5 μl | 2.5 μl |
| Total | 25.0 μl | 25.0 μl |

**Table 10** PCR conditions

| Temperature | Time | Cycles | Comment |
|---|---|---|---|
| 50 °C | 30 min | 1 | Reverse Transcription |
| 96 °C | 2 min | 1 | Hot Start |
| 96 °C | 20 s | | Denaturation |
| 60 °C | 45 s | 35 | Annealing |
| 68 °C | 45 s | | Elongation |
| 68 °C | 7 min | 1 | Final Elongation |
| 4 °C | 0 to ∞ | 1 | Storage |

## Gel electrophoresis

Gel electrophoresis is used to separate the amplicons generated in the PCR step by size. The gel electrophoresis was performed with the FlashGel system by Lonza. This system uses ready to use 1.2 % agarose gel cassettes were 2 μl sample mixed with 0.5 μl 5× Loading Dye are loaded. The prepared gels have 13 slots. The best way to check the lengths of the amplicons is load all attenuation site specific amplicons together on one gel. The electrophoresis was performed 6 min at 250 V.

## Library preparation and sequencing

Residuals of the PCR reaction mix are washed away in a PCR clean-up step using a commercial kit. Afterwards the Index-PCR was performed to give each amplicon an individual index. The use of indexes enables parallel sequencing of multiple samples. As a control, the Sequencing Control for Amplicon sequencing with MiSeq generated from PCV2 and Nextera XT adapter (SC-AMPX) is used. A second PCR Clean-Up may be performed after the Index-PCR. It is optional to perform a second FlashGel electrophoresis to check PCR and Clean-Up. The resulting amplicons with index were quantified by Quant-iT PicoGreen dsDNA Assay Kit and diluted on 0.8 nM. After pooling the different normalized libraries, the sequencing with MiSeq was performed with a sequencing kit which provides a minimum of 390 bp sequencing length.

**Table 11** Variants of TDV and positive control EC TDV

| Sample | Amplicon | Reference Position | Genome Position | Wild type | Variation | Expected frequency |
|--------|----------|--------------------|-----------------|-----------|-----------|--------------------|
| TDV-1, TDV-2, TDV-4 | ATT1 | 27 nt | 57 nt | C | T | ≥95% |
| | ATT2 | 104 nt | 2579 nt | G | A | |
| | ATT3 | 140 nt | 5270 nt | A | T | |
| TDV-3 | ATT1 | 27 nt | 57 nt | C | T | |
| | ATT2 | 104 nt | 2573 nt | G | A | |
| | ATT3 | 140 nt | 5264 nt | A | T | |
| EC TDV | ATT1 | 27 nt | 57 nt | C | T | ≥95% |
| | | 195 nt | 225 nt | A | T | ≈ 5 % |
| | ATT3 | 140 nt | 5270 nt | A | T | ≥95% |
| | | 261 nt | 5391 nt | C | T | ≈ 5 % |
| SC-AMPX | / | 12 nt | 288 nt | C | T | 21.6 % ± 2.2 % |
| | / | 60 nt | 336 nt | C | G | |

### Results

### Primer specificity

The fusion primers allow the specific amplification of each attenuation site (ATT1, ATT2 and ATT3) in a sufficient manner (Figure 1). To analyze samples of the same or different serotype in one sequencing reaction each amplicon was amplified by Index-PCR with individual indexing primers. The fusion primers with overhanging adapter sequences amplified the attenuation sites without the production of unspecific fragments (Figure 2). Additionally they enabled the indexing by PCR.

**Table 12** Length of the 3 attenuation specific amplicons

| Amplicon | Length | | | |
|----------|--------------------------|----------------------|------------------------------|-----------------------------------|
| | Sequence of interest | With specific primers | With fusion primers (+67 bp) | Final indexed library (+69 bp) |
| ATT1 | 217 bp | 257 bp | 324 bp | 393 bp |
| ATT2 | 224 bp | 266 bp | 333 bp | 402 bp |
| ATT3 | 298 bp | 341 bp | 408 bp | 477 bp |

### Specificity - RT-PCR with fusion primers

This test was done to verify that the initial PCR-assay with fusion primers is able to amplify the target regions (ATT1, ATT2 and ATT3) of each TDV serotype in a sufficient manner. The expected amplicons generate a distinct fragment at FlashGel.

**Table 13** Results of specificity test - RT-PCR with fusion primers

| Sample | Fragment | Demand | Result |
|--------|----------|--------|--------|
| NTC | ATT1 | No amplicon | No amplicon |
| NEC | | No amplicon | No amplicon |
| TDV-1 | | About 324 bp | About 324 bp |
| TDV-2 | | About 324 bp | About 324 bp |
| TDV-3 | | About 324 bp | About 324 bp |
| TDV-4 | | About 324 bp | About 324 bp |
| NTC | ATT2 | No amplicon | No amplicon |
| NEC | | No amplicon | No amplicon |
| TDV-1 | | About 333 bp | About 333 bp |
| TDV-2 | | About 333 bp | About 333 bp |
| TDV-3 | | About 333 bp | About 333 bp |
| TDV-4 | | About 333 bp | About 333 bp |
| NTC | ATT3 | No amplicon | No amplicon |
| NEC | | No amplicon | No amplicon |
| TDV-1 | | About 408 bp | About 408 bp |
| TDV-2 | | About 408 bp | About 408 bp |
| TDV-3 | | About 408 bp | About 408 bp |
| TDV-4 | | About 408 bp | About 408 bp |

## Specificity - Index-PCR

This test was done to verify that the PCR-assay for indexing the amplicons is able to produce specific amplicons with the index sequence to differentiate the samples.

**Table 14** Results of specificity test - Index-PCR

| Sample | Fragment | Demand | Result |
|--------|----------|--------|--------|
| TDV-1 | ATT1 | About 393 bp | About 393 bp |
| TDV-2 | | About 393 bp | About 393 bp |
| TDV-3 | | About 393 bp | About 393 bp |
| TDV-4 | | About 393 bp | About 393 bp |
| TDV-1 | ATT2 | About 402 bp | About 402 bp |
| TDV-2 | | About 402 bp | About 402 bp |
| TDV-3 | | About 402 bp | About 402 bp |
| TDV-4 | | About 402 bp | About 402 bp |
| TDV-1 | ATT3 | About 477 bp | About 477 bp |
| TDV-2 | | About 477 bp | About 477 bp |
| TDV-3 | | About 477 bp | About 477 bp |
| TDV-4 | | About 477 bp | About 477 bp |

## Specificity - Sequencing

All attenuation specific amplicons of each TDV serotype were able to detect and match with 100 % to the given references (defined variations not regarded).

**Table 15** Summary of TDV sequencing results

| Criteria | Value (average ± standard deviation) |
|---|---|
| Percentage of mapped reads. | 95.9 % ± 1.9 % |
| Frequency of ATT variants | 99.7 % ± 0.1 % |
| Sequencing failure | 0.1 % ± 0.1 % |

**Table 16** Results of specificity test - Defined variations at sequencing

| Sample | Fragment | Demand | Result |
|---|---|---|---|
| TDV-1 | ATT1 | Position 57 nt: C → T, ≥ 95 %* | C → T, ≥ 99.71 % |
| TDV-2 | | | C → T, ≥ 99.70 % |
| TDV-3 | | | C → T, ≥ 99.81 % |
| TDV-4 | | | C → T, ≥ 99.73 % |
| TDV-1 | ATT2 | Position 2579 nt (TDV-3: 2573 nt): G → A, ≥ 95 %* | G → A, ≥ 99.78 % |
| TDV-2 | | | G → A, ≥ 99.84 % |
| TDV-3 | | | G → A, ≥ 99.82 % |
| TDV-4 | | | G → A, ≥ 99.79 % |
| TDV-1 | ATT3 | Position 5270 nt (TDV-3: 5264 nt): A → T, ≥ 95 %* | A → T, ≥ 99.68 % |
| TDV-2 | | | A → T, ≥ 99.41 % |
| TDV-3 | | | A → T, ≥ 99.67 % |
| TDV-4 | | | A → T, ≥ 99.75 % |
| * Wild type (reference) is attenuated to (→) Called Base (Variation) with a frequency of at least 95 % | | | |

## Sensitivity

The purpose of this experiment is to confirm the sensitivity of the assay to detect the attenuating genotype when the proportion of attenuating genotype is 5% or less. A variation of ± 4 % is accepted.

**Table 17** Extraction Control (EC) sample for TDV

| Sample | Characteristics | Concentration |
|---|---|---|
| EC TDV | Mixture of TDV-1, -2 and -3 spiked with 5% TDV-4; different mixtures because of no direct correlation between virus and genome titer | TDV-1, -2 & -3: $1.49{\times}10^{8}$ $^{FFU}/_{ml}$<br>eachTDV-4: $5.24{\times}10^{6}$ $^{FFU}/_{ml}$ |

To confirm the sensitivity of the assay to detect an attenuation genotype a mixture of TDV-1, - TDV-2 and TDV-3 was spiked with TDV-4. TDV-4 has additional variations at position 225 nt (T instead of A, within the amplicon also comprising ATT1) and 5391 nt (T instead of C, within the amplicon also comprising ATT3), which can be used to differentiate TDV-4 from the other serotypes. The percentage was calculated using the virus titer.

The RNA extraction was performed as described previously. The RT-PCR was performed as described previously. The following Library preparation (Clean-Up 1, Index-PCR and second Clean-Up) and sequencing were only done with the amplicons of ATT1 and ATT3 as described previously.

**Table 18** Results of sensitivity test - Recovery of TDV-4

| Sample | Fragment | Demand | Result | Coverage |
|---|---|---|---|---|
| | | | | |
| EC TDV* | ATT1 Position 225 nt | A → T, ≈ 5 %** | 7.42 % | 48739 reads |
| | | | 6.63 % | 48737 reads |
| | | | 6.89 % | 48590 reads |
| EC TDV* | ATT3 Position 5391 nt | C → T, ≈ 5 %** | 6.42 % | 36805 reads |
| | | | 6.38 % | 47099 reads |
| | | | 6.25 % | 48345 reads |
| * EC TDV was sequenced 3-times to generate more data to define the SST-criteria when it is used as control (EC, AC, and PSC) <br> ** Reference is mixed with TDV-4 to (→) Called Base (Variation) with a frequency of ≈ 5 % <br> *** Average ± standard deviation | | | | |

**Table 19** Results of sensitivity test - Recovery of TDV-4 at EC TDV at different Coverages

| Ø Coverage | Fragment | Demand | Results* | Variance |
|---|---|---|---|---|
| 20284 reads | ATT1 Position 225 nt | A → T**, 7.42 %; 6.63 %; 6.89 %; (The Ø Coverage of 48689 reads was used as initial value to compare with the lower coverages (Table 18)) | 7.52 %; 6.51 %; 6.93 % | 0,12% |
| 12169 reads | | | 7.43 %; 6.92 %; 7.07 % | 0,29% |
| 10040 reads | | | 7.58 %; 6.83 %; 7.53 % | 0,64% |
| 5018 reads | | | 7.90 %; 7.45 %; 8.37 % | 1,48% |
| 2006 reads | | | 7.94 %; 7.17 %; 8.35 % | 1,46% |
| 1000 reads | | | 8.77 %; 7.22 %; 8.38 % | 1,49% |
| 495 reads | | | 8.55 %; 7.55 %; 5.93 % | 1,13% |
| 193 reads | | | 5.82 %; 9.79 %; 7.93 % | 3,16% |
| 91 reads | | | 9.19 %; 2.15 %; 10.22 % | 3,33% |
| 20123 reads | ATT3 Position 5391 nt | C → T**, 6.42 %; 6.38 %; 6.25 %; (The Ø Coverage of 44083 reads was used as initial value to compare with the lower coverages (Table 18)) | 6.22 %; 6.31 %; 6.25 % | 0,20% |
| 12071 reads | | | 6.19 %; 6.26 %; 6.29 % | 0,23% |
| 9948 reads | | | 6.32 %; 6.08 %; 6.21 % | 0,30% |
| 4980 reads | | | 6.30 %; 6.27 %; 6.49 % | 0,24% |
| 1983 reads | | | 5.21 %; 7.10 %; 6.51 % | 1,21% |
| 991 reads | | | 4.77 %; 7.06 %; 7.45 % | 1,65% |
| 492 reads | | | 5.43 %; 6.31 %; 5.78 % | 0,99% |
| 194 reads | | | 3.22 %; 5.94 %; 2.16 % | 4,09% |
| 94 reads | | | 4.49 %; 8.98 %; 2.22 % | 4,03% |
| * EC TDV was sequenced 3-times to generate more data to define the SST-criteria when it is used as control (EC, AC, and PSC) <br> ** Reference is mixed with TDV-4 to (→) Called Base (Variation) with a frequency of ≈ 5 % | | | | |

0 shows that the variants at position 225 nt and 5391 nt were detectable at a coverage of ca. 100 reads. To determine the minimum number of reads that may be used to minimalize errors, coverage of between 91 and 20284 reads were compared to the average coverage of a run with 48689 reads for ATT1; and coverage of between 94 and 20123 reads were compared to the average coverage from a run with 44083 reads for ATT3. The difference between the average coverage from 48689 reads (ATT1) and 44083 reads (ATT3) was > 3 % for the 91 and 94 reads, respectively. Ideally, the differences should be ≤ 0.5 % (native failure: 0.1 % (Table 15) + standard deviation of triple sequencing: 0.4 % (Table 18)).

With coverage of at least 12,000 reads a difference of <0.3 % could be reached. In consequence, the minimal coverage to analyze a sample or TDV control to achieve this detection limit should be 12,000 reads.

### Summary of results

The specificity of the primer was confirmed by combination of RT-PCR, Index-PCR and sequencing. It could be shown a high specificity regarding the appropriate related attenuation at each primer pair. The size and the sequence of the resulting PCR fragments comply with the expectations.

Each attenuation could be detected in all four serotypes with a ratio of 99 % or greater (Table 16) by analysis of the sequencing results with SeqMan NGen (DNASTAR, Version 13.0.0 (360)) and SeqMan Pro (DNASTAR, Version 13.0.0 (361), 422). Therefore, it can be concluded that the parameter specificity of the assay is fulfilled.

It could be shown that a titer of $1 \times 10^7 \, ^{\text{FFU}}/_{\text{ml}}$ is enough material to generate the needed amount of ATT-specific amplicons. Samples with lower titers are not expected.

Different annealing temperatures had no influence on the RT-PCR. It could be concluded that the primer binding site is very specific and stringent.

Thus it was demonstrated that the method is robust and suitable as routine sequencing test to confirm the attenuating genotype.

### Claims

1. A method for determining the proportion of an attenuated genotype of a live, attenuated flavivirus in a formulation comprising (i) at least one live, attenuated Dengue virus having a nucleotide sequence comprising an attenuated-type nucleotide at at least one attenuation locus, and (ii) and optionally at least one further Dengue virus having a nucleotide sequence comprising a nucleotide other than the attenuated-type nucleotide at the at least one attenuation locus, wherein the method comprises the steps:

   a) purifying Dengue virus nucleic acids from said formulation;
   b) preparing double-stranded DNA templates from said Dengue virus nucleic acids;
   c) amplifying said viral nucleic acids of a) or said double-stranded DNA templates of b) by polymerase chain reaction (PCR) by using a primer pair comprising sequences specific for a Dengue virus sequence comprising the at least one attenuation locus such that PCR products comprising the at least one attenuation locus are generated;
   d) sequencing said PCR products by next generation sequencing by synthesis; and
   e) determining the proportion of the attenuated genotype of a live, attenuated Dengue virus in the formulation from the ratio of

      (i) the number of sequence reads in which the nucleotide at the at least one attenuation locus is identical to the nucleotide of the live, attenuated flavivirus at the at least one attenuation locus to
      (ii) the total number of sequence reads,

   wherein the live, attenuated dengue virus comprises three attenuation loci at positions 57, 2579 and 5270, wherein the sequence numbering relates to the nucleotide sequence of dengue virus type 2 according to SEQ ID NO: 2 and the primer pair of step c) is selected from the group consisting of:

      i) a forward primer comprising SEQ ID NO: 11, or a variant thereof having at least 95% sequence identity and a reverse primer comprising SEQ ID NO: 12, or a variant thereof having at least 95% sequence identity;
      ii) a forward primer comprising SEQ ID NO: 13, or a variant thereof having at least 95% sequence identity and a reverse primer pair comprising SEQ ID NO: 14, or a variant thereof having at least 95% sequence identity; and
      iii) a forward primer comprising SEQ ID NO: 15, or a variant thereof having at least 95% sequence identity and a reverse primer comprising SEQ ID NO: 16, or a variant thereof having at least 95% sequence identity.

2. The method of claim 1, wherein prior to step a) the at least one live, attenuated virus is grown in Vero cells.

3. The method of claims 1 or 2, wherein the final concentration of each of the forward and reverse primer in the amplifying step c) is about 0.1 to about 0.3 $\mu$M.

4. The method of any one of claims 1 to 3, wherein the PCR reaction comprises a denaturing step at about 96°C for about 20s, an annealing step at about 60°C for about 45s, and an extension step at about 68°C for about 45s.

5. The method of any one of claims 1 to 4, wherein after step c) one or more of the following steps is/are performed:

(i) purifying the PCR products;
(ii) performing an Index-PCR reaction on the PCR products of step c) using forward and reverse index primer pairs thereby obtaining indexed PCR products; and/or
(iii) purifying the PCR products of step (ii).

6. The method of any one of claims 1 to 5, wherein the sequencing step is performed on the indexed PCR products of claim 5, alternative (ii).

7. The method of any one of claims 1 to 6, wherein the method comprises the following steps:

a) purifying Dengue virus nucleic acids from said formulation;
b) preparing double-stranded DNA templates from said Dengue virus nucleic acids;
c) amplifying said viral nucleic acids of a) or said double-stranded DNA templates of b) by polymerase chain reaction (PCR) by using a primer pair specific for a Dengue virus sequence comprising the at least one attenuation locus such that PCR products comprising the attenuation locus are generated;
d) purifying the PCR products;
e) performing an Index-PCR reaction on the PCR products of step c) using forward and reverse index primer pairs thereby obtaining indexed PCR products; and/or
f) purifying the indexed PCR products;
g) sequencing the indexed PCR products by next generation sequencing by synthesis; and
h) determining the proportion of the attenuated genotype of a live, attenuated Dengue virus in the formulation from the ratio of

(i) the number of sequence reads in which the nucleotide at the at least one attenuation locus is identical to the nucleotide of the live, attenuated Dengue virus at the at least one attenuation locus to
(ii) the total number of sequence reads.

8. The method of any one of claims 5 to 7, wherein before the sequencing step) bridge amplification PCR using oligonucleotides complementary to the 5' end of the indexed PCR product is carried out, wherein the oligonucleotides are immobilized on a support.

9. The method of any one of claims 1 to 8, wherein the next generation sequencing by synthesis is carried out on a platform selected from any one of Roche 454, Illumina MiSeq, Illumina HiSeq, and Life Technologies SOLiD4.

10. The method of claim 9, wherein the platform is Illumina MiSeq.

11. The method of any one of claims 1 to 10, wherein the next generation sequencing by synthesis is reversible terminator chemistry sequencing.

12. The method of any one of claims 1 to 11, wherein at least 12000 sequence reads, preferably at least 20000 sequence reads, more preferably at least 30000 sequence reads are analysed.

13. The method according to any one of claims 1 to 12, wherein the Dengue virus formulation comprises a tetravalent dengue vaccine.

14. The method of any one of claims 1 to 13, wherein the Dengue virus formulation comprises one or more live, attenuated flavivirus selected from TDV-1 having the nucleotide sequence set forth in SEQ ID NO: 1, TDV-2 having the nucleotide sequence set forth in SEQ ID NO: 2, TDV-3 having the nucleotide sequence set forth in SEQ ID NO: 3 and TDV-4 having the nucleotide sequence set forth in SEQ ID NO: 4, preferably the Dengue virus formulation comprises each of TDV-1 to TDV-4.

15. Use of the method of any one of claims 1 to 14 in the quality control of vaccines containing at least one live, attenuated Dengue virus.

**16.** A method for the quality control of vaccines comprising at least one live, attenuated Dengue virus comprising performing the method of any one of claims 1 to 14 and performing a quality control step, wherein the vaccine is accepted if the proportion of the at least one live, attenuated virus in the vaccine is at least 90%, preferably at least 95 %, more preferably at least 97% and most preferably at least 99%.

**17.** The method for the quality control of vaccines of claim 16, further comprising performing at least one further method selected from the group consisting of immunofocus assay, visual inspection, determination of reconstitution time or resuspendability of lyophilisates, sterility test, test for bacterial endotoxins, determination of host cell DNA, determination of pH, colorimetric determination of water, determination of osmolality, determination of the content of one or more excipient(s).

**Patentansprüche**

**1.** Verfahren zum Bestimmen des Anteils eines abgeschwächten Genotyps eines lebenden, abgeschwächten Flavivirus in einer Formulierung, umfassend (i) mindestens ein lebendes, abgeschwächtes Dengue-Virus mit einer Nukleotidsequenz, umfassend ein Nukleotid vom Abschwächungstyp an mindestens einem Abschwächungslokus, und (ii) gegebenenfalls mindestens ein weiteres Dengue-Virus mit einer Nukleotidsequenz, umfassend ein von dem Nukleotid vom Abschwächungstyp verschiedenes Nukleotid an dem mindestens einen Abschwächungslokus, wobei das Verfahren die folgenden Schritte umfasst:

a) Aufreinigen der Dengue-Virus-Nukleinsäuren aus der Formulierung;
b) Herstellen doppelsträngiger DNA-Matrizen aus den Dengue-Virus-Nukleinsäuren;
c) Amplifizieren der viralen Nukleinsäuren aus a) oder der doppelsträngigen DNA-Matrizen aus b) durch Polymerase-Kettenreaktion (PCR) derart unter Verwendung eines Primerpaares, umfassend Sequenzen, die für eine den mindestens einen Abschwächungslokus umfassende Dengue-Virussequenz spezifisch sind, dass den mindestens einen Abschwächungslokus umfassende PCR-Produkte erzeugt werden;
d) Sequenzieren der PCR-Produkte mittels Next-Generation-Sequenzieren durch Synthese; und
e) Bestimmen des Anteils des abgeschwächten Genotyps eines lebenden, abgeschwächten Dengue-Virus in der Formulierung anhand des Verhältnisses von

(i) der Anzahl von Sequenz-Reads, bei denen das Nukleotid an dem mindestens einen Abschwächungslokus mit dem Nukleotid des lebenden, abgeschwächten Flavivirus an dem mindestens einen Abschwächungslokus identisch ist, zu
(ii) der Gesamtanzahl der Sequenz-Reads,

wobei das lebende, abgeschwächte Dengue-Virus drei Abschwächungslokusse an den Positionen 57, 2579 und 5270 umfasst, wobei sich die Sequenznummerierung auf die Nukleotidsequenz des Dengue-Virus Typ 2 gemäß SEQ ID NO: 2 bezieht und das Primerpaar aus Schritt c) ausgewählt ist aus der Gruppe, bestehend aus:

i) einem Vorwärtsprimer, umfassend SEQ ID NO: 11 oder einer Variante davon mit mindestens 95%iger Sequenzidentität, und einem Rückwärtsprimer, umfassend SEQ ID NO: 12 oder einer Variante davon mit mindestens 95%iger Sequenzidentität;
ii) einem Vorwärtsprimer, umfassend SEQ ID NO: 13 oder einer Variante davon mit mindestens 95%iger Sequenzidentität, und einem Rückwärtsprimerpaar, umfassend SEQ ID NO: 14 oder einer Variante davon mit mindestens 95%iger Sequenzidentität; und
iii) einem Vorwärtsprimer, umfassend SEQ ID NO: 15 oder einer Variante davon mit mindestens 95%iger Sequenzidentität, und einem Rückwärtsprimer, umfassend SEQ ID NO: 16 oder einer Variante davon mit mindestens 95%iger Sequenzidentität.

**2.** Verfahren nach Anspruch 1, wobei vor Schritt a) das mindestens eine lebende, abgeschwächte Virus in Vero-Zellen gezüchtet wird.

**3.** Verfahren nach Anspruch 1 oder 2, wobei die Endkonzentration jedes Vorwärts- und Rückwärtsprimers in Amplifizierschritt c) etwa 0,1 bis etwa 0,3 μM beträgt.

**4.** Verfahren nach einem der Ansprüche 1 bis 3, wobei die PCR-Reaktion einen Denaturierungsschritt bei etwa 96 °C über etwa 20 s, einen Annealing-Schritt bei etwa 60 °C über etwa 45 s und einen Extensionsschritt bei etwa 68 °C über etwa 45 s umfasst.

5. Verfahren nach einem der Ansprüche 1 bis 4, wobei nach Schritt c) einer oder mehrere der folgenden Schritte durchgeführt wird/werden:

   (i) Aufreinigen der PCR-Produkte;
   (ii) Durchführen einer Index-PCR-Reaktion an den PCR-Produkten aus Schritt c) unter Verwendung von Vorwärts- und Rückwärts-Indexprimerpaaren, wodurch indexierte PCR-Produkte erhalten werden; und/oder
   (iii) Aufreinigen der PCR-Produkte aus Schritt (ii).

6. Verfahren nach einem der Ansprüche 1 bis 5, wobei der Sequenzierschritt mit den indizierten PCR-Produkten nach Anspruch 5, Alternative (ii), durchgeführt wird.

7. Verfahren nach einem der Ansprüche 1 bis 6, wobei das Verfahren die folgenden Schritte umfasst:

   a) Aufreinigen der Dengue-Virus-Nukleinsäuren aus der Formulierung;
   b) Herstellen doppelsträngiger DNA-Matrizen aus den Dengue-Virus-Nukleinsäuren;
   c) Amplifizieren der viralen Nukleinsäuren aus a) oder der doppelsträngigen DNA-Matrizen aus b) durch Polymerase-Kettenreaktion (PCR) derart unter Verwendung eines Primerpaares, das für eine den mindestens einen Abschwächungslokus umfassende Dengue-Virussequenz spezifisch ist, dass den Abschwächungslokus umfassende PCR-Produkte erzeugt werden;
   d) Aufreinigen der PCR-Produkte;
   e) Durchführen einer Index-PCR-Reaktion an den PCR-Produkten aus Schritt c) unter Verwendung von Vorwärts- und Rückwärts-Indexprimerpaaren, wodurch indexierte PCR-Produkte erhalten werden; und/oder
   f) Aufreinigen der indexierten PCR-Produkte;
   g) Sequenzieren der indexierten PCR-Produkte mittels Next-Generation-Sequenzieren durch Synthese; und
   h) Bestimmen des Anteils des abgeschwächten Genotyps eines lebenden, abgeschwächten Dengue-Virus in der Formulierung anhand des Verhältnisses von

   (i) der Anzahl von Sequenz-Reads, bei denen das Nukleotid an dem mindestens einen Abschwächungslokus mit dem Nukleotid des lebenden, abgeschwächten Dengue-Virus an dem mindestens einen Abschwächungslokus identisch ist, zu
   (ii) der Gesamtanzahl der Sequenz-Reads.

8. Verfahren nach einem der Ansprüche 5 bis 7, wobei vor dem Sequenzierschritt Brückenamplifikations-PCR unter Verwendung von zu dem 5'-Ende des indexierten PCR-Produkts komplementären Oligonukleotiden durchgeführt wird, wobei die Oligonukleotide auf einem Träger immobilisiert sind.

9. Verfahren nach einem der Ansprüche 1 bis 8, wobei das Next-Generation-Sequenzieren durch Synthese auf einer Plattform durchgeführt wird, ausgewählt aus einer der von Roche 454, Illumina MiSeq, Illumina HiSeq oder Life Technologies SOLiD4.

10. Verfahren nach Anspruch 9, wobei es sich bei der Plattform um Illumina MiSeq handelt.

11. Verfahren nach einem der Ansprüche 1 bis 10, wobei es sich bei dem Next-Generation-Sequenzieren durch Synthese um reversibles Terminatorchemie-Sequenzieren handelt.

12. Verfahren nach einem der Ansprüche 1 bis 11, wobei mindestens 12.000 Sequenz-Reads, vorzugsweise mindestens 20.000 Sequenz-Reads, mehr bevorzugt mindestens 30.000 Sequenz-Reads analysiert werden.

13. Verfahren nach einem der Ansprüche 1 bis 12, wobei die Dengue-Virusformulierung einen tetravalenten Dengue-Impfstoff umfasst.

14. Verfahren nach einem der Ansprüche 1 bis 13, wobei die Dengue-Virusformulierung ein oder mehrere lebende, abgeschwächte Flaviviren umfasst, ausgewählt aus TDV-1 mit der in SEQ ID NO: 1 angegebenen Nukleotidsequenz, TDV-2 mit der in SEQ ID NO: 2 angegebenen Nukleotidsequenz, TDV-3 mit der in SEQ ID NO: 3 angegebenen Nukleotidsequenz und TDV-4 mit der in SEQ ID NO: 4 angegebenen Nukleotidsequenz, wobei die Dengue-Virus-Formulierung vorzugsweise jedes von TDV-1 bis TDV-4 umfasst.

**15.** Verwendung des Verfahrens nach einem der Ansprüche 1 bis 14 bei der Qualitätskontrolle von Impfstoffen, die mindestens ein lebendes, abgeschwächtes Dengue-Virus enthalten.

**16.** Verfahren zur Qualitätskontrolle von mindestens ein lebendes, abgeschwächtes Dengue-Virus umfassenden Impfstoffen, umfassend Durchführen des Verfahrens nach einem der Ansprüche 1 bis 14 und Durchführen eines Qualitätskontrollschritts, wobei der Impfstoff akzeptiert wird, wenn der Anteil des mindestens einen lebenden, abgeschwächten Virus in dem Impfstoff mindestens 90 %, vorzugsweise mindestens 95 %, mehr bevorzugt mindestens 97 % und am meisten bevorzugt mindestens 99 % beträgt.

**17.** Verfahren zur Qualitätskontrolle von Impfstoffen nach Anspruch 16, ferner umfassend Durchführen mindestens eines weiteren Verfahrens, ausgewählt aus der Gruppe, bestehend aus Immunofokus-Test, Sichtprüfung, Bestimmung der Rekonstitutionszeit oder Resuspendierbarkeit von Lyophilisaten, Sterilitätsprüfung, Prüfung auf bakterielle Endotoxine, Bestimmung der Wirtszellen-DNA, Bestimmung des pH-Wertes, kolorimetrischer Bestimmung von Wasser, Bestimmung der Osmolalität, Bestimmung des Gehalts eines oder mehrerer Hilfsstoffe.

**Revendications**

**1.** Procédé de détermination de la proportion d'un génotype atténué d'un flavivirus vivant atténué dans une formulation comprenant (i) au moins un virus de la dengue vivant atténué présentant une séquence nucléotidique comprenant un nucléotide de type atténué à au moins un locus d'atténuation ; et (ii) éventuellement au moins un autre virus de la dengue présentant une séquence nucléotidique comprenant un nucléotide autre que le nucléotide de type atténué à l'au moins un locus d'atténuation, dans lequel le procédé comprend les étapes :

a) de purification d'acides nucléiques du virus de la dengue à partir de ladite formulation ;
b) de préparation de matrices d'ADN double brin à partir desdits acides nucléiques du virus de la dengue ;
c) d'amplification desdits acides nucléiques viraux de a) ou desdites matrices d'ADN double brin de b) par réaction en chaîne par polymérase (PCR) à l'aide d'une paire d'amorces comprenant des séquences spécifiques d'une séquence du virus de la dengue comprenant l'au moins un locus d'atténuation, de sorte que des produits de PCR comprenant l'au moins un locus d'atténuation soient générés ;
d) de séquençage desdits produits de PCR par séquençage de nouvelle génération par synthèse ; et
e) de détermination de la proportion du génotype atténué d'un virus de la dengue vivant atténué dans la formulation à partir du rapport entre

(i) le nombre de lectures de séquences dans lesquelles le nucléotide à l'au moins un locus d'atténuation est identique au nucléotide du flavivirus vivant atténué à l'au moins un locus d'atténuation et
(ii) le nombre total de lectures de séquences,

dans lequel le virus de la dengue vivant atténué comprend trois loci d'atténuation aux positions 57, 2579 et 5270, dans lequel la numérotation de séquences se rapporte à la séquence nucléotidique du virus de la dengue de type 2 selon SEQ ID N° : 2 et la paire d'amorces de l'étape c) est choisie dans le groupe constitué de :

i) une amorce sens comprenant SEQ ID N° : 11, ou une variante de celle-ci présentant une identité de séquence d'au moins 95 % et une amorce antisens comprenant SEQ ID N° : 12, ou une variante de celle-ci présentant une identité de séquence d'au moins 95 % ;
ii) une amorce sens comprenant SEQ ID N° : 13, ou une variante de celle-ci présentant une identité de séquence d'au moins 95 %, et une paire d'amorces antisens comprenant SEQ ID N° : 14, ou une variante de celle-ci présentant une identité de séquence d'au moins 95 % ; et
iii) une amorce sens comprenant SEQ ID N° : 15, ou une variante de celle-ci présentant une identité de séquence d'au moins 95 %, et une amorce antisens comprenant SEQ ID N° : 16, ou une variante de celle-ci présentant une identité de séquence d'au moins 95 %.

**2.** Procédé selon la revendication 1, dans lequel, avant l'étape a), l'au moins un virus vivant atténué est cultivé dans des cellules Vero.

**3.** Procédé selon les revendications 1 ou 2, dans lequel la concentration finale de chacune des amorces sens et antisens lors de l'étape c) d'amplification est d'environ 0,1 à environ 0,3 μM.

**4.** Procédé selon l'une quelconque des revendications 1 à 3, dans lequel la réaction PCR comprend une étape de dénaturation à environ 96 °C pendant environ 20 s, une étape d'hybridation à environ 60 °C pendant environ 45 s et une étape d'extension à environ 68 °C pendant environ 45 s.

**5.** Procédé selon l'une quelconque des revendications 1 à 4, dans lequel, après l'étape c), une ou plusieurs des étapes suivantes est/sont réalisée(s) :

(i) purification des produits de PCR ;
(ii) réalisation d'une réaction PCR d'indexation sur les produits de PCR de l'étape c) à l'aide de paires d'amorces d'indexation sens et antisens, de manière à obtenir des produits de PCR indexés ; et/ou
(iii) purification des produits de PCR de l'étape (ii).

**6.** Procédé selon l'une quelconque des revendications 1 à 5, dans lequel l'étape de séquençage est réalisée sur les produits de PCR indexés de la revendication 5, alternative (ii).

**7.** Procédé selon l'une quelconque des revendications 1 à 6, dans lequel le procédé comprend les étapes suivantes :

a) purification d'acides nucléiques du virus de la dengue à partir de ladite formulation ;
b) préparation de matrices d'ADN double brin à partir desdits acides nucléiques du virus de la dengue ;
c) amplification desdits acides nucléiques viraux de a) ou desdites matrices d'ADN double brin de b) par réaction en chaîne par polymérase (PCR) à l'aide d'une paire d'amorces spécifique d'une séquence du virus de la dengue comprenant l'au moins un locus d'atténuation, de sorte que des produits de PCR comprenant le locus d'atténuation soient générés ;
d) purification des produits de PCR ;
e) réalisation d'une réaction PCR d'indexation sur les produits de PCR de l'étape c) à l'aide de paires d'amorces d'indexation sens et antisens, de manière à obtenir des produits de PCR indexés ; et/ou
f) purification des produits de PCR indexés ;
g) séquençage des produits de PCR indexés par séquençage de nouvelle génération par synthèse ; et
h) détermination de la proportion du génotype atténué d'un virus de la dengue vivant atténué dans la formulation à partir du rapport entre

(i) le nombre de lectures de séquences dans lesquelles le nucléotide à l'au moins un locus d'atténuation est identique au nucléotide du virus de la dengue vivant atténué à l'au moins un locus d'atténuation et
(ii) le nombre total de lectures de séquences.

**8.** Procédé selon l'une quelconque des revendications 5 à 7, dans lequel, avant l'étape de séquençage, une PCR d'amplification en pont est réalisée à l'aide d'oligonucléotides complémentaires de l'extrémité 5' du produit de PCR indexé, dans lequel les oligonucléotides sont immobilisés sur un support.

**9.** Procédé selon l'une quelconque des revendications 1 à 8, dans lequel le séquençage de nouvelle génération par synthèse est réalisé sur une plateforme choisie parmi l'une quelconque de Roche 454, Illumina MiSeq, Illumina HiSeq et Life Technologies SOLiD4.

**10.** Procédé selon la revendication 9, dans lequel la plateforme est Illumina MiSeq.

**11.** Procédé selon l'une quelconque des revendications 1 à 10, dans lequel le séquençage de nouvelle génération par synthèse est un séquençage par chimie de terminateur réversible.

**12.** Procédé selon l'une quelconque des revendications 1 à 11, dans lequel au moins 12 000 lectures de séquences, de préférence au moins 20 000 lectures de séquences, et plus préférablement au moins 30 000 lectures de séquences, sont analysées.

**13.** Procédé selon l'une quelconque des revendications 1 à 12, dans lequel la formulation du virus de la dengue comprend un vaccin tétravalent contre la dengue.

**14.** Procédé selon l'une quelconque des revendications 1 à 13, dans lequel la formulation du virus de la dengue comprend un ou plusieurs flavivirus vivants atténués choisis parmi TDV-1 présentant la séquence nucléotidique indiquée dans SEQ ID N° : 1, TDV-2 présentant la séquence nucléotidique indiquée dans SEQ ID N° : 2, TDV-3 présentant la séquence nucléotidique indiquée dans SEQ **ID** N° : 3 et TDV-4 présentant la séquence nucléotidique indiquée dans SEQ ID N° : 4, de préférence la formulation du virus de la dengue comprend chacun de TDV-1 à TDV-4.

15. Utilisation du procédé selon l'une quelconque des revendications 1 à 14 pour le contrôle de la qualité de vaccins contenant au moins un virus de la dengue vivant atténué.

16. Procédé de contrôle de la qualité de vaccins comprenant au moins un virus de la dengue vivant atténué, comprenant la réalisation du procédé selon l'une quelconque des revendications 1 à 14 et la réalisation d'une étape de contrôle de la qualité, dans lequel le vaccin est accepté si la proportion de l'au moins un virus vivant atténué dans le vaccin est d'au moins 90 %, de préférence d'au moins 95 %, plus préférablement d'au moins 97 % et de manière préférée entre toutes d'au moins 99 %.

17. Procédé de contrôle de la qualité de vaccins selon la revendication 16, comprenant également la réalisation d'au moins un autre procédé choisi dans le groupe constitué d'un essai d'immunofocus, d'une inspection visuelle, d'une détermination du temps de reconstitution ou de la capacité de remise en suspension de lyophilisats, d'un essai de stérilité, d'un essai de détection d'endotoxines bactériennes, d'une détermination d'ADN de cellules hôtes, d'une détermination du pH, d'une détermination colorimétrique de l'eau, d'une détermination de l'osmolalité, d'une détermination de la teneur en un ou plusieurs excipient(s).

Fig. 1

**Fig. 2**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3892737 A
- WO 9837911 A
- WO 03101397 A
- WO 2007021672 A
- WO 2008007021 A
- WO 2008047023 A
- WO 2008065315 A
- WO 01060847 A2
- WO 2014150939 A2
- WO 2017179017 A1

**Non-patent literature cited in the description**

- **BUTRAPET et al.** *J. Virol.*, 2000, vol. 74 (7), 3011-3019
- **BUTRAPET et al.** *J. Virol.*, 2000, vol. 74, 3011-3019
- **BHAMARAPRAVATI et al.** *Bulletin of the World Health Organization*, 1987, vol. 65 (2), 189-195
- **HUANG et al.** *PLoS Negl Trop Dis*, 2013, vol. 7 (5), e2243
- **DURBIN et al.** *Journal of Infectious Diseases*, 2013, vol. 207, 957-965